# EUROPEAN PATENT APPLICATION

(11) **EP 4 458 863 A1**
(43) Date of publication of application: **06.11.2024**
(21) Application number: 22915130.3
(22) Date of filing: 30.12.2022
(51) Int. Cl.: C07K 19/00, A61K 38/26, A61P 3/04

(54) **FUSION PROTEIN OF GLP-1 AND GDF15 AND USE THEREOF**

(30) Priority: 31.12.2021 CN 202111678485
(71) Applicant: Sunshine Lake Pharma Co., Ltd., Dongguan, Guangdong 523000 (CN)
(72) Inventor: YAN, Jiangyu, Dongguan, Guangdong 523871 (CN); XIAO, Lin, Dongguan, Guangdong 523871 (CN); ZENG, Junnan, Dongguan, Guangdong 523871 (CN); GONG, Qingwei, Dongguan, Guangdong 523871 (CN); CHENG, Xiaoli, Dongguan, Guangdong 523871 (CN); LIU, Xiaoying, Dongguan, Guangdong 523871 (CN); LI, Wenjia, Dongguan, Guangdong 523871 (CN)
(74) Representative: Hoffmann Eitle
(86) International application number: PCT/CN2022/143676
(87) International publication number: WO 2023/125881

(57) **Abstract**

The present invention relates to a fusion protein of GLP-1 and GDF15 and the use thereof. The fusion protein comprises a first polypeptide fragment, a second polypeptide fragment and a third polypeptide fragment which are sequentially connected from an amino terminal to a carboxyl terminal; and the three polypeptide fragments sequentially comprise a GLP-1 polypeptide, an immunoglobulin Fc region and a GDF15 active domain. The fusion protein provided has a GLP-1 and GDF15 dual-target activity. The fusion protein as a whole has excellent physicochemical properties and stability, and the two target molecules of GLP-1 and GDF15 have a good activity and pharmacokinetic balance, facilitating the exertion of a dual-target effect.

## Description

### FIELD OF THE INVENTION

The invention relates to the technical field of biomedicine, in particular to a fusion protein of GLP-1 and GDF15 and use thereof.

### BACKGROUND ART

Growth differentiation factor 15 (GDF15), also known as macrophage inhibitory cytokine-1 (MIC-1), placental transforming growth factor-β (PTGF-β), placental bone morphogenetic factor (PLAB), prostate-derived factor (PDF), non-steroidal anti-inflammatory drug-activated gene (NAG-1), is a branch member of the transforming growth factor (TGF-β) superfamily. The mature GDF15 polypeptide contains 112 amino acids, while the biologically active GDF15 circulating in vivo is a homodimeric protein (24.5kD) formed by two polypeptides through interchain disulfide bonds.

It has been reported that elevated circulating levels of GDF15 is associated with reduced food intake and weight loss in patients with advanced cancer (Johnen H et al, Nat Med, 2007). Furthermore, both transgenic mice expressing high GDF15 as well as mice administered recombinant GDF15 exhibited reduced body weight and food intake, and had improved levels of glucose tolerance (Xiong Y et al., Sci Transl Med, 2017), indicating that GDF15 has the potential to treat diseases such as obesity, type II diabetes (T2D), and nonalcoholic steatohepatitis (NASH).

Glucagon-like peptide-1 (GLP-1) is a peptide hormone secreted by intestinal L cells, which can lower blood glucose by promoting insulin secretion, and can also reduce body weight by suppressing appetite and delaying gastric emptying. The main bioactive fragment of GLP-1 is a peptide fragment of 30 or 31 amino acids derived from post-translational processing of glucagon peptide (amino acids 7-36 or 7-37 of GLP-1). Based on the above biological effects, GLP-1 analogues have been successfully used in clinical practice for the treatment of type II diabetes and obesity.

Although GDF15 and GLP-1 can reduce body weight and improve metabolic syndrome by suppressing appetite, the pathways of these two molecules may not overlap in terms of mechanism of action. Therefore, improved technical solutions are needed to exert the role of the two molecules at the same time, to achieve better weight loss and improve metabolic syndrome efficacy.

### SUMMARY

The purpose of the present invention is to provide a fusion protein of GLP-1 and GDF15 and use thereof, the present invention constructs a fusion protein with GLP-1 and GDF15 dual-target activity, the fusion protein has excellent physicochemical properties and stability, and the two target molecules of GLP-1 and GDF15 have good balance of activity and pharmacokinetics, which is conducive to the exertion of dual-target effect.

For this purpose, the first aspect of the present invention provides a fusion protein comprising: a) a first polypeptide fragment, which comprises a glucagon-like peptide-1 (GLP-1) polypeptide; b) a second polypeptide fragment, which comprises an immunoglobulin Fc region; c) a third polypeptide fragment, which comprises a growth differentiation factor 15 (GDF15) active domain; and the first polypeptide fragment, the second polypeptide fragment and the third polypeptide fragment are sequentially linked along the direction from amino terminus to carboxyl terminus through a linker peptide.

Further, the GLP-1 polypeptide comprises one of the following amino acid sequences: SEQ ID NO:1, SEQ ID NO:2, SEQ ID NO:3, or SEQ ID NO:4; or,
the GLP-1 polypeptide comprises an amino acid sequence having at least 85%, 90%, 95%, or 99% sequence identity to one of the following sequences: SEQ ID NO:1, SEQ ID NO:2, SEQ ID NO:3, or SEQ ID NO:4; or,
the GLP-1 polypeptide has no more than 6, 5, 4, 3, 2, 1 mutations relative to SEQ ID NO:1, SEQ ID NO:2, SEQ ID NO:3 or SEQ ID NO:4, while retaining the basic biological activity of the pre-mutation polypeptide.

In some embodiments, the GLP-1 polypeptide comprises one of the following mutations, or a combination of two or more mutations at different positions: A8G, G22E, I29V, A30E, A30Q, R36G. Wherein, the position of the mutation site follows the position numbering in SEQ ID NO: 1 (GLP-1 (1-37)).

In some embodiments, the GLP-1 polypeptide comprises one of the following mutations: A8G, G22E, R36G; or A8G, G22E, A30E, R36G; or A8G, G22E, A30Q, R36G; or A8G, G22E, I29V, A30Q, R36G.

In a certain embodiment, the GLP-1 polypeptide with mutations comprises the following amino acid sequences: SEQ ID NO: 5, SEQ ID NO: 6, SEQ ID NO: 7 or SEQ ID NO: 8.

In another embodiment, the GLP-1 polypeptide comprises an amino acid sequence having at least 85%, 90%, 95% or 99% sequence identity to one of the following sequences: SEQ ID NO: 5, SEQ ID NO: 6, SEQ ID NO: 7 or SEQ ID NO: 8.

**Table 1 Glucagon-like peptide-1 (GLP-1) polypeptide**

| SEQ ID NO: | Sequences | Note |
|---|---|---|
| 1 | HDEFERHAEGTFTSDVSSYLEGQAAKEFIAWL VKGRG | GLP-1(1-37) |
| 2 | HAEGTFTSDVSSYLEGQAAKEFIAWLVKGRG | GLP-1(7-37) |
| 3 | HDEFERHAEGTFTSDVSSYLEGQAAKEFIAWL VKGR | GLP-1(1-36) |
| 4 | HAEGTFTSDVSSYLEGQAAKEFIAWLVKGR | GLP-1(7-36) |
| 5 | HGEGTFTSDVSSYLEEQAAKEFIAWLVKGGG | GLP-1(7-37, A8G, G22E, R36G) |
| 6 | HGEGTFTSDVSSYLEEQAAKEFIEWLVKGGG | GLP-1(7-37, A8G, G22E, A30E, R36G) |
| 7 | HGEGTFTSDVSSYLEEQAAKEFIQWLVKGGG | GLP-1(7-37, A8G, G22E, A30Q, R36G) |
| 8 | HGEGTFTSDVSSYLEEQAAKEFVQWLVKGGG | GLP-1(7-37, A8G, G22E, I29V, A30Q, R36G) |

| | | |
|---|---|---|
| Note: In Table 1, the numbering of amino acid positions follows the position numbers in SEQ ID NO: 1 (GLP-1 (1-37)), that is, the first amino acid at the N-terminus of SEQ ID NO: 1 is the first position, and the numbers are sequentially numbered in the direction of the C-terminus. | | |

In some embodiments, the immunoglobulin Fc region is IgG Fc, and further, it is selected from one of IgG1 Fc, IgG2, IgG3 Fc, and IgG4 Fc.

In some embodiments, the immunoglobulin Fc region is a human immunoglobulin Fc region. In some embodiments, the immunoglobulin Fc region is a human IgG Fc, such as a human IgG1 Fc or a human IgG4 Fc, etc.

Further, the immunoglobulin Fc region is a native immunoglobulin Fc region or an immunoglobulin Fc region variant.

In an embodiment, the immunoglobulin Fc region comprises an amino acid sequence of SEQ ID NO: 9 or SEQ ID NO: 10; or, the immunoglobulin Fc region comprises an amino acid sequence having at least 85%, 90%, 95% or 99% sequence identity to one of the following sequences: SEQ ID NO: 9, SEQ ID NO: 10; or, the immunoglobulin Fc region has no more than 6, 5, 4, 3, 2, 1 mutations relative to SEQ ID NO: 9, SEQ ID NO: 10, while retaining the basic biological activity of the pre-mutation polypeptide.

In some embodiments, the immunoglobulin Fc region variant comprises the following mutation: an amino acid at position 356 of IgG Fc is substituted with an amino acid other than D, E and C according to EU numbering. For example, the amino acid at position 356 of IgG Fc is substituted with one of the followings: G, S, A, T, V, N, L, I, Q, Y, F, H, P, M, K, R.

In some embodiments, the immunoglobulin Fc region variant comprises the following mutation: an amino acid at position 439 of IgG Fc is substituted with an amino acid other than R, H, K, and C according to EU numbering. For example, the amino acid at position 439 of IgG Fc is substituted with one of the followings: G, S, A, T, V, D, N, L, I, E, Q, Y, F, P, M.

In some embodiments, the immunoglobulin Fc region variant comprises the following mutations in the IgG Fc according to EU numbering: one of E356G, E356S, E356A, E356T, E356V, E356N, E356L, E356I, E356Q, E356Y, E356F, E356H, E356P, E356M, E356K, E356R, and/or one of K439G, K439S, K439A, K439T, K439V, K439D, K439N, K439L, K439I, K439E, K439Q, K439Y, K439F, K439H, K439P, K439M.

In preferred embodiments, the immunoglobulin Fc region variant comprises the following mutations in the IgG Fc according to EU numbering: one of E356K, E356R, E356Q, E356A, E356N, and/or one of K439D, K439E, K439Q, K439A, K439N.

In some embodiments, the immunoglobulin Fc region variant further comprises the following mutations: the amino acids at positions 234 and 235 of IgG Fc are substituted with AA, and/or the amino acid at position 447 is deleted according to EU numbering. For example, for IgG1 Fc, the following mutations are also comprised: L234A and L235A, and/or the amino acid at position 447 is deleted; for IgG4 Fc, the following mutations are also comprised: F234A and L235A, and/or the amino acid at position 447 is deleted.

In a certain embodiment, the immunoglobulin Fc region variant has only one of the following amino acid substitutions in the IgG Fc according to EU numbering:
amino acid substitution at position 356 according to EU numbering;
amino acid substitution at position 439 according to EU numbering;
amino acid substitutions at position 356 and 439 according to EU numbering;
amino acid substitutions at positions 234 and 235 according to EU numbering;
amino acid substitutions at positions 356, 234 and 235 according to EU numbering;
amino acid substitutions at positions 439, 234 and 235 according to EU numbering;
amino acid substitutions at positions 356, 439, 234 and 235 according to EU numbering;
the amino acid substitutions described at the respective positions have the meanings described in the present invention.

In a certain embodiment, the amino acid sequence of the immunoglobulin Fc region variant comprises one of the following sequences: SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37.

In another embodiment, the immunoglobulin Fc region variant comprises an amino acid sequence having at least 85%, 90%, 95% or 99% sequence identity to one of the following sequences: SEQ ID NO: 11-37.

**Table 2 Immunoglobulin Fc region**

| SEQ ID NO: | Sequences | Note |
|---|---|---|
| 9 | GPSVFLFPPKPKDTLMISRTPEVTCVVVDVSQEDPEVQFNWYV DGVEVHNAKTKPREEQFNSTYRVVSVLTVLHQDWLNGKEYKC KVSNKGLPSSIEKTISKAKGQPREPQVYTLPPSQEEMTKNQVSL TCLVKGFYPSDIAVEWESNGQPENNYKTTPPVLDSDGSFFLYSR LTVDKSRWQEGNVFSCSVMHEALHNHYTQKSLSLSLG | Fc (G) |
| 10 | GGPSVFLFPPKPKDTLMISRTPEVTCVVVDVSQEDPEVQFNWY VDGVEVHNAKTKPREEQFNSTYRVVSVLTVLHQDWLNGKEYK CKVSNKGLPSSIEKTISKAKGQPREPQVYTLPPSQEEMTKNQVS LTCLVKGFYPSDIAVEWESNGQPENNYKTTPPVLDSDGSFFLYS RLTVDKSRWQEGNVFSCSVMHEALHNHYTQKSLSLSLG | Fc (GG) |
| 11 | EAAGGPSVFLFPPKPKDTLMISRTPEVTCVVVDVSQEDPEVQFN WYVDGVEVHNAKTKPREEQFNSTYRVVSVLTVLHQDWLNGK EYKCKVSNKGLPSSIEKTISKAKGQPREPQVYTLPPSQEEMTKN QVSLTCLVKGFYPSDIAVEWESNGQPENNYKTTPPVLDSDGSFF LYSRLTVDKSRWQEGNVFSCSVMHEALHNHYTQKSLSLSLG | Fc (AA) |
| 12 | APEAAGGPSVFLFPPKPKDTLMISRTPEVTCVVVDVSQEDPEVQ FNWYVDGVEVHNAKTKPREEQFNSTYRVVSVLTVLHQDWLNG KEYKCKVSNKGLPSSIEKTISKAKGQPREPQVYTLPPSQEEMTK NQVSLTCLVKGFYPSDIAVEWESNGQPENNYKTTPPVLDSDGSF FLYSRLTVDKSRWQEGNVFSCSVMHEALHNHYTQKSLSLSLG | Fc (APEAA ) |
| 13 | GPSVFLFPPKPKDTLMISRTPEVTCVVVDVSQEDPEVQFNWYV | Fc (G, |
| | DGVEVHNAKTKPREEQFNSTYRVVSVLTVLHQDWLNGKEYKC KVSNKGLPSSIEKTISKAKGQPREPQVYTLPPSQEEMTKNQVSL TCLVKGFYPSDIAVEWESNGQPENNYKTTPPVLDSDGSFFLYSR LTVDKSRWQEGNVFSCSVMHEALHNHYTQESLSLSLG | K439E) |
| 14 | GGPSVFLFPPKPKDTLMISRTPEVTCVVVDVSQEDPEVQFNWY VDGVEVHNAKTKPREEQFNSTYRVVSVLTVLHQDWLNGKEYK CKVSNKGLPSSIEKTISKAKGQPREPQVYTLPPSQEEMTKNQVS LTCLVKGFYPSDIAVEWESNGQPENNYKTTPPVLDSDGSFFLYS RLTVDKSRWQEGNVFSCSVMHEALHNHYTQESLSLSLG | Fc (GG, K439E) |
| 15 | EAAGGPSVFLFPPKPKDTLMISRTPEVTCVVVDVSQEDPEVQFN WYVDGVEVHNAKTKPREEQFNSTYRVVSVLTVLHQDWLNGK EYKCKVSNKGLPSSIEKTISKAKGQPREPQVYTLPPSQEEMTKN QVSLTCLVKGFYPSDIAVEWESNGQPENNYKTTPPVLDSDGSFF LYSRLTVDKSRWQEGNVFSCSVMHEALHNHYTQESLSLSLG | Fc (AA, K439E) |
| 16 | APEAAGGPSVFLFPPKPKDTLMISRTPEVTCVVVDVSQEDPEVQ FNWYVDGVEVHNAKTKPREEQFNSTYRVVSVLTVLHQDWLNG KEYKCKVSNKGLPSSIEKTISKAKGQPREPQVYTLPPSQEEMTK NQVSLTCLVKGFYPSDIAVEWESNGQPENNYKTTPPVLDSDGSF FLYSRLTVDKSRWQEGNVFSCSVMHEALHNHYTQESLSLSLG | Fc (APEAA, K439E) |
| 17 | GPSVFLFPPKPKDTLMISRTPEVTCVVVDVSQEDPEVQFNWYV DGVEVHNAKTKPREEQFNSTYRVVSVLTVLHQDWLNGKEYKC KVSNKGLPSSIEKTISKAKGQPREPQVYTLPPSQEEMTKNQVSL TCLVKGFYPSDIAVEWESNGQPENNYKTTPPVLDSDGSFFLYSR LTVDKSRWQEGNVFSCSVMHEALHNHYTQDSLSLSLG | Fc (G, K439D) |
| 18 | GGPSVFLFPPKPKDTLMISRTPEVTCVVVDVSQEDPEVQFNWY VDGVEVHNAKTKPREEQFNSTYRVVSVLTVLHQDWLNGKEYK CKVSNKGLPSSIEKTISKAKGQPREPQVYTLPPSQEEMTKNQVS LTCLVKGFYPSDIAVEWESNGQPENNYKTTPPVLDSDGSFFLYS RLTVDKSRWQEGNVFSCSVMHEALHNHYTQDSLSLSLG | Fc (GG, K439D) |
| 19 | EAAGGPSVFLFPPKPKDTLMISRTPEVTCVVVDVSQEDPEVQFN WYVDGVEVHNAKTKPREEQFNSTYRVVSVLTVLHQDWLNGK EYKCKVSNKGLPSSIEKTISKAKGQPREPQVYTLPPSQEEMTKN QVSLTCLVKGFYPSDIAVEWESNGQPENNYKTTPPVLDSDGSFF LYSRLTVDKSRWQEGNVFSCSVMHEALHNHYTQDSLSLSLG | Fc (AA, K439D) |
| 20 | APEAAGGPSVFLFPPKPKDTLMISRTPEVTCVVVDVSQEDPEVQ FNWYVDGVEVHNAKTKPREEQFNSTYRVVSVLTVLHQDWLNG KEYKCKVSNKGLPSSIEKTISKAKGQPREPQVYTLPPSQEEMTK NQVSLTCLVKGFYPSDIAVEWESNGQPENNYKTTPPVLDSDGSF FLYSRLTVDKSRWQEGNVFSCSVMHEALHNHYTQDSLSLSLG | Fc (APEAA, K439D) |
| 21 | GPSVFLFPPKPKDTLMISRTPEVTCVVVDVSQEDPEVQFNWYV DGVEVHNAKTKPREEQFNSTYRVVSVLTVLHQDWLNGKEYKC KVSNKGLPSSIEKTISKAKGQPREPQVYTLPPSQKEMTKNQVSL TCLVKGFYPSDIAVEWESNGQPENNYKTTPPVLDSDGSFFLYSR LTVDKSRWQEGNVFSCSVMHEALHNHYTQKSLSLSLG | Fc (G, E356K) |
| 22 | GGPSVFLFPPKPKDTLMISRTPEVTCVVVDVSQEDPEVQFNWY VDGVEVHNAKTKPREEQFNSTYRVVSVLTVLHQDWLNGKEYK CKVSNKGLPSSIEKTISKAKGQPREPQVYTLPPSQKEMTKNQVS LTCLVKGFYPSDIAVEWESNGQPENNYKTTPPVLDSDGSFFLYS RLTVDKSRWQEGNVFSCSVMHEALHNHYTQKSLSLSLG | Fc (GG, E356K) |
| 23 | EAAGGPSVFLFPPKPKDTLMISRTPEVTCVVVDVSQEDPEVQFN WYVDGVEVHNAKTKPREEQFNSTYRVVSVLTVLHQDWLNGK EYKCKVSNKGLPSSIEKTISKAKGQPREPQVYTLPPSQKEMTKN QVSLTCLVKGFYPSDIAVEWESNGQPENNYKTTPPVLDSDGSFF LYSRLTVDKSRWQEGNVFSCSVMHEALHNHYTQKSLSLSLG | Fc (AA, E356K) |
| 24 | APEAAGGPSVFLFPPKPKDTLMISRTPEVTCVVVDVSQEDPEVQ FNWYVDGVEVHNAKTKPREEQFNSTYRVVSVLTVLHQDWLNG KEYKCKVSNKGLPSSIEKTISKAKGQPREPQVYTLPPSQKEMTK NQVSLTCLVKGFYPSDIAVEWESNGQPENNYKTTPPVLDSDGSF FLYSRLTVDKSRWQEGNVFSCSVMHEALHNHYTQKSLSLSLG | Fc (APEAA, E356K) |
| 25 | GPSVFLFPPKPKDTLMISRTPEVTCVVVDVSQEDPEVQFNWYV DGVEVHNAKTKPREEQFNSTYRVVSVLTVLHQDWLNGKEYKC KVSNKGLPSSIEKTISKAKGQPREPQVYTLPPSQREMTKNQVSL TCLVKGFYPSDIAVEWESNGQPENNYKTTPPVLDSDGSFFLYSR LTVDKSRWQEGNVFSCSVMHEALHNHYTQKSLSLSLG | Fc (G, E356R) |
| 26 | GGPSVFLFPPKPKDTLMISRTPEVTCVVVDVSQEDPEVQFNWY VDGVEVHNAKTKPREEQFNSTYRVVSVLTVLHQDWLNGKEYK CKVSNKGLPSSIEKTISKAKGQPREPQVYTLPPSQREMTKNQVS LTCLVKGFYPSDIAVEWESNGQPENNYKTTPPVLDSDGSFFLYS RLTVDKSRWQEGNVFSCSVMHEALHNHYTQKSLSLSLG | Fc (GG, E356R) |
| 27 | EAAGGPSVFLFPPKPKDTLMISRTPEVTCVVVDVSQEDPEVQFN WYVDGVEVHNAKTKPREEQFNSTYRVVSVLTVLHQDWLNGK EYKCKVSNKGLPSSIEKTISKAKGQPREPQVYTLPPSQREMTKN QVSLTCLVKGFYPSDIAVEWESNGQPENNYKTTPPVLDSDGSFF LYSRLTVDKSRWQEGNVFSCSVMHEALHNHYTQKSLSLSLG | Fc (AA, E356R) |
| 28 | APEAAGGPSVFLFPPKPKDTLMISRTPEVTCVVVDVSQEDPEVQ FNWYVDGVEVHNAKTKPREEQFNSTYRVVSVLTVLHQDWLNG KEYKCKVSNKGLPSSIEKTISKAKGQPREPQVYTLPPSQREMTK NQVSLTCLVKGFYPSDIAVEWESNGQPENNYKTTPPVLDSDGSF | Fc (APEAA, E356R) |
| | FLYSRLTVDKSRWQEGNVFSCSVMHEALHNHYTQKSLSLSLG | |
| 29 | EAAGGPSVFLFPPKPKDTLMISRTPEVTCVVVDVSQEDPEVQFN WYVDGVEVHNAKTKPREEQFNSTYRVVSVLTVLHQDWLNGK EYKCKVSNKGLPSSIEKTISKAKGQPREPQVYTLPPSQEEMTKN QVSLTCLVKGFYPSDIAVEWESNGQPENNYKTTPPVLDSDGSFF LYSRLTVDKSRWQEGNVFSCSVMHEALHNHYTQQSLSLSLG | Fc (AA, K439Q) |
| 30 | APEAAGGPSVFLFPPKPKDTLMISRTPEVTCVVVDVSQEDPEVQ FNWYVDGVEVHNAKTKPREEQFNSTYRVVSVLTVLHQDWLNG KEYKCKVSNKGLPSSIEKTISKAKGQPREPQVYTLPPSQEEMTK NQVSLTCLVKGFYPSDIAVEWESNGQPENNYKTTPPVLDSDGSF FLYSRLTVDKSRWQEGNVFSCSVMHEALHNHYTQQSLSLSLG | Fc (APEAA, K439Q) |
| 31 | APEAAGGPSVFLFPPKPKDTLMISRTPEVTCVVVDVSQEDPEVQ FNWYVDGVEVHNAKTKPREEQFNSTYRVVSVLTVLHQDWLNG KEYKCKVSNKGLPSSIEKTISKAKGQPREPQVYTLPPSQEEMTK NQVSLTCLVKGFYPSDIAVEWESNGQPENNYKTTPPVLDSDGSF FLYSRLTVDKSRWQEGNVFSCSVMHEALHNHYTQNSLSLSLG | Fc (APEAA, K439N) |
| 32 | APEAAGGPSVFLFPPKPKDTLMISRTPEVTCVVVDVSQEDPEVQ FNWYVDGVEVHNAKTKPREEQFNSTYRVVSVLTVLHQDWLNG KEYKCKVSNKGLPSSIEKTISKAKGQPREPQVYTLPPSQEEMTK NQVSLTCLVKGFYPSDIAVEWESNGQPENNYKTTPPVLDSDGSF FLYSRLTVDKSRWQEGNVFSCSVMHEALHNHYTQASLSLSLG | Fc (APEAA, K439A) |
| 33 | APEAAGGPSVFLFPPKPKDTLMISRTPEVTCVVVDVSQEDPEVQ FNWYVDGVEVHNAKTKPREEQFNSTYRVVSVLTVLHQDWLNG KEYKCKVSNKGLPSSIEKTISKAKGQPREPQVYTLPPSQQEMTK NQVSLTCLVKGFYPSDIAVEWESNGQPENNYKTTPPVLDSDGSF FLYSRLTVDKSRWQEGNVFSCSVMHEALHNHYTQKSLSLSLG | Fc (APEAA, E356Q) |
| 34 | APEAAGGPSVFLFPPKPKDTLMISRTPEVTCVVVDVSQEDPEVQ FNWYVDGVEVHNAKTKPREEQFNSTYRVVSVLTVLHQDWLNG KEYKCKVSNKGLPSSIEKTISKAKGQPREPQVYTLPPSQNEMTK NQVSLTCLVKGFYPSDIAVEWESNGQPENNYKTTPPVLDSDGSF FLYSRLTVDKSRWQEGNVFSCSVMHEALHNHYTQKSLSLSLG | Fc (APEAA, E356N) |
| 35 | APEAAGGPSVFLFPPKPKDTLMISRTPEVTCVVVDVSQEDPEVQ FNWYVDGVEVHNAKTKPREEQFNSTYRVVSVLTVLHQDWLNG KEYKCKVSNKGLPSSIEKTISKAKGQPREPQVYTLPPSQAEMTK NQVSLTCLVKGFYPSDIAVEWESNGQPENNYKTTPPVLDSDGSF FLYSRLTVDKSRWQEGNVFSCSVMHEALHNHYTQKSLSLSLG | Fc (APEAA, E356A) |
| 36 | AESKYGPPCPPCPAPEAAGGPSVFLFPPKPKDTLMISRTPEVTCV VVDVSQEDPEVQFNWYVDGVEVHNAKTKPREEQFNSTYRVVS VLTVLHQDWLNGKEYKCKVSNKGLPSSIEKTISKAKGQPREPQ | Fc (Hinge, AA, |
| | VYTLPPSQEEMTKNQVSLTCLVKGFYPSDIAVEWESNGQPENN YKTTPPVLDSDGSFFLYSRLTVDKSRWQEGNVFSCSVMHEALH NHYTQESLSLSLG | K439E) |
| 37 | APEAAGGPSVFLFPPKPKDTLMISRTPEVTCVVVDVSHEDPEVK FNWYVDGVEVHNAKTKPREEQYNSTYRVVSVLTVLHQDWLN GKEYKCKVSNKALPAPIEKTISKAKGQPREPQVYTLPPSRDELT KNQVSLTCLVKGFYPSDIAVEWESNGQPENNYKTTPPVLDSDGS FFLYSKLTVDKSRWQQGNVFSCSVMHEALHNHYTQESLSLSPG | IgG1 Fc (APEAA, K439E) |
| 82 | EAAGGPSVFLFPPKPKDTLMISRTPEVTCVVVDVSQEDPEVQFN WYVDGVEVHNAKTKPREEQFNSTYRVVSVLTVLHQDWLNGK EYKCKVSNKGLPSSIEKTISKAKGQPREPQVYTLPPSQEEMTKN QVSLTCLVKGFYPSDIAVEWESNGQPENNYKTTPPVLDSDGSFQ LESRLTVDKSRWQEGNVFSCSVMHEALHNHYTQKSLSLSLG | Fc (AA, F405Q/ Y407E) |

| | | |
|---|---|---|
| Note: In Table 2, the numbering of amino acid positions is in accordance with the EU numbering system. | | |

Further, the GDF15 active domain comprises an amino acid sequence selected from one of the following groups:
SEQ ID NO: 38; or,
an amino acid sequence having at least 85%, 90%, 95% or 99% sequence identity to SEQ ID NO: 38, or,
1-14 amino acid truncations at the N-terminus of SEQ ID NO: 38, and/or SEQ ID NO: 38 having no more than 5, 4, 3, 2, 1 mutations while retaining the basic biological activity of the pre-mutation polypeptide.

Further, the number of amino acid truncations at the N-terminus of SEQ ID NO: 38 is 1, 2, 3, 4, 5, 6, 7, 8, 9, 10, 11, 12, 13 or 14.

Further, the position of amino acid mutation in SEQ ID NO: 38 is selected from one, two or three of the followings: position 5, position 6, position 21, position 26, position 30, position 47, position 54, position 55, position 57, position 67, position 69, position 81, position 94, position 107.

Further, the amino acid mutation in SEQ ID NO: 38 is selected from one, any two or three of the different positions of the followings: D5E, H6D, H6E, R21Q, R21H, D26E, A30S, A47D, A54S, A55E, M57T, R67Q, K69R, A81S, T94E, K107Q.

In some embodiments, the GDF15 active domain comprises the amino acid sequence shown in SEQ ID NO: 38.

In other embodiments, the GDF15 active domain comprises the following sequence: an amino acid sequence truncated by 1-14 amino acids at the N-terminus of SEQ ID NO:38.

In still other embodiments, the GDF15 active domain comprises the following sequence: an amino acid sequence having no more than 5, 4, 3, 2, 1 mutations in SEQ ID NO: 38; the amino acid mutations in SEQ ID NO: 38 have the meanings described in the present invention.

In yet other embodiments, the GDF15 active domain comprises the following sequence: an amino acid sequence with 1-14 amino acid truncations at the N-terminus of SEQ ID NO: 38 and no more than 5, 4, 3, 2, 1 mutations in SEQ ID NO: 38; the amino acid substitutions in SEQ ID NO: 38 have the meanings described in the present invention.

In a certain embodiment, the GDF15 active domain comprises one of the following sequences: SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58.

In another embodiment, the GDF15 active domain comprises an amino acid sequence having at least 85%, 90%, 95% or 99% sequence identity to one of the following sequences: SEQ ID NO: 38-58.

**Table 3 Growth differentiation factor 15 (GDF15) active domain**

| SEQ ID NO: | Sequences | Note |
|---|---|---|
| 38 | ARNGDHCPLGPGRCCRLHTVRASLEDLGWADWVLSPR EVQVTMCIGACPSQFRAANMHAQIKTSLHRLKPDTVPAP CCVPASYNPMVLIQKTDTGVSLQTYDDLLAKDCHCI | WT |
| 39 | GDHCPLGPGRCCRLHTVRASLEDLGWADWVLSPREVQ VTMCIGACPSQFRAANMHAQIKTSLHRLKPDTVPAPCCV | ΔN3 |
| | PASYNPMVLIQKTDTGVSLQTYDDLLAKDCHCI | |
| 40 | DHCPLGPGRCCRLHTVRASLEDLGWADWVLSPREVQVT MCIGACPSQFRAANMHAQIKTSLHRLKPDTVPAPCCVPA SYNPMVLIQKTDTGVSLQTYDDLLAKDCHCI | ΔN4 |
| 41 | CRLHTVRASLEDLGWADWVLSPREVQVTMCIGACPSQF RAANMHAQIKTSLHRLKPDTVPAPCCVPASYNPMVLIQ KTDTGVSLQTYDDLLAKDCHCI | ΔN14 |
| 42 | EHCPLGPGRCCRLHTVRASLEDLGWADWVLSPREVQVT MCIGACPSQFRAANMHAQIKTSLHRLKPDTVPAPCCVPA SYNPMVLIQKTDTGVSLQTYDDLLAKDCHCI | ΔN4, D5E |
| 43 | DCPLGPGRCCRLHTVRASLEDLGWADWVLSPREVQVT MCIGACPSQFRAANMHAQIKTSLHRLKPDTVPAPCCVPA SYNPMVLIQKTDTGVSLQTYDDLLAKDCHCI | ΔN5, H6D |
| 44 | DECPLGPGRCCRLHTVRASLEDLGWADWVLSPREVQVT MCIGACPSQFRAANMHAQIKTSLHRLKPDTVPAPCCVPA SYNPMVLIQKTDTGVSLQTYDDLLAKDCHCI | ΔN4, H6E |
| 45 | ECPLGPGRCCRLHTVRASLEDLGWADWVLSPREVQVT MCIGACPSQFRAANMHAQIKTSLHRLKPDTVPAPCCVPA SYNPMVLIQKTDTGVSLQTYDDLLAKDCHCI | ΔN5, H6E |
| 46 | DHCPLGPGRCCRLHTVQASLEDLGWADWVLSPREVQV TMCIGACPSQFRAANMHAQIKTSLHRLKPDTVPAPCCVP ASYNPMVLIQKTDTGVSLQTYDDLLAKDCHCI | ΔN4, R21Q |
| 47 | DHCPLGPGRCCRLHTVHASLEDLGWADWVLSPREVQV TMCIGACPSQFRAANMHAQIKTSLHRLKPDTVPAPCCVP ASYNPMVLIQKTDTGVSLQTYDDLLAKDCHCI | ΔN4, R21H |
| 48 | DHCPLGPGRCCRLHTVRASLEELGWADWVLSPREVQVT MCIGACPSQFRAANMHAQIKTSLHRLKPDTVPAPCCVPA SYNPMVLIQKTDTGVSLQTYDDLLAKDCHCI | ΔN4, D26E |
| 49 | DHCPLGPGRCCRLHTVRASLEELGWADWVLSPREVQVT MCIGACPSQFRAANMHAQIKTSLHRLRPDTVPAPCCVPA SYNPMVLIQKTDTGVSLQTYDDLLAKDCHCI | ΔN4, D26E, K69R |
| 50 | DHCPLGPGRCCRLHTVRASLEDLGWSDWVLSPREVQVT MCIGACPSQFRAANMHAQIKTSLHRLKPDTVPAPCCVPA SYNPMVLIQKTDTGVSLQTYDDLLAKDCHCI | ΔN4, A30S |
| 51 | DHCPLGPGRCCRLHTVRASLEDLGWADWVLSPREVQVT MCIGDCPSQFRAANMHAQIKTSLHRLKPDTVPAPCCVPA SYNPMVLIQKTDTGVSLQTYDDLLAKDCHCI | ΔN4, A47D |
| 52 | DHCPLGPGRCCRLHTVRASLEDLGWADWVLSPREVQVT MCIGACPSQFRSANMHAQIKTSLHRLKPDTVPAPCCVPA SYNPMVLIQKTDTGVSLQTYDDLLAKDCHCI | ΔN4, A54S |
| 53 | DHCPLGPGRCCRLHTVRASLEDLGWADWVLSPREVQVT MCIGACPSQFRAENMHAQIKTSLHRLKPDTVPAPCCVPA SYNPMVLIQKTDTGVSLQTYDDLLAKDCHCI | ΔN4, A55E |
| 54 | DHCPLGPGRCCRLHTVRASLEDLGWADWVLSPREVQVT MCIGACPSQFRAANTHAQIKTSLHRLKPDTVPAPCCVPA SYNPMVLIQKTDTGVSLQTYDDLLAKDCHCI | ΔN4, M57T |
| 55 | DHCPLGPGRCCRLHTVRASLEDLGWADWVLSPREVQVT MCIGACPSQFRAANMHAQIKTSLHQLKPDTVPAPCCVPA SYNPMVLIQKTDTGVSLQTYDDLLAKDCHCI | ΔN4, R67Q |
| 56 | DHCPLGPGRCCRLHTVRASLEDLGWADWVLSPREVQVT MCIGACPSQFRAANMHAQIKTSLHRLKPDTVPAPCCVPS SYNPMVLIQKTDTGVSLQTYDDLLAKDCHCI | ΔN4, A81S |
| 57 | DHCPLGPGRCCRLHTVRASLEDLGWADWVLSPREVQVT MCIGACPSQFRAANMHAQIKTSLHRLKPDTVPAPCCVPA SYNPMVLIQKTDEGVSLQTYDDLLAKDCHCI | ΔN4, T94E |
| 58 | DHCPLGPGRCCRLHTVRASLEDLGWADWVLSPREVQVT MCIGACPSQFRAANMHAQIKTSLHRLKPDTVPAPCCVPA SYNPMVLIQKTDTGVSLQTYDDLLAQDCHCI | ΔN4, K107Q |

| | | |
|---|---|---|
| Note: In Table 3, the numbering method for indicating amino acid positions is as follows: the first amino acid at the N-terminus of SEQ ID NO: 38 is the first position, and the numbers are sequentially numbered in the direction of the C-terminus. | | |

In some embodiments, the first polypeptide fragment and the second polypeptide fragment are connected through a first peptide linker, and/or the second polypeptide fragment and the third polypeptide fragment are connected through a second peptide linker.

Further, each of the first peptide linker and the second peptide linker is independently selected from one or a combination of flexible peptide linkers, rigid peptide linkers.

In some embodiments, the first peptide linker and the second peptide linker are each independently selected from one or a combination of (G₄X)ₙ, (X'P)ₘ, (EAAAK)ₚ, G_{q}, wherein each of n, m, p, q is independently selected from an integer of 1-10, X is serine (S) or alanine (A), and X' is alanine (A), lysine (K) or glutamic acid (E).

In a certain embodiment, the first peptide linker and/or the second peptide linker comprise a combination of (G₄X)ₙ and (X'P)ₘ, wherein n, m, X, X' have the meanings described in the present invention.

In a certain embodiment, the first peptide linker and/or the second peptide linker comprise (G₄X)ₙ₁-(X'P)ₘ-(G₄X)ₙ₂, wherein each of n₁, m, n₂ is independently selected from an integer of 1-10, X is S or A, and X' is A, K or E.

In a certain embodiment, the first peptide linker and/or the second peptide linker comprise (G₄X)ₙ₁-(X'P)ₘ-(G₄X)ₙ₂, wherein n₁ is 2, m is 10, n₂ is 2.

In another embodiment, the first peptide linker and/or the second peptide linker comprise a combination of G_{q} and (X'P)ₘ, wherein q, m, X' have the meanings described in the present invention.

In a certain embodiment, the first peptide linker and/or the second peptide linker comprise G_{q1}-(X'P)ₘ-G_{q2}, wherein each of q1, m and q2 is independently selected from an integer of 1-10, and X' is A, K or E.

In a certain embodiment, the first peptide linker and/or the second peptide linker comprise G_{q1}-(X'P)ₘ-G_{q2}, wherein q₁ is 4, m is 10, q₂ is 4.

In another embodiment, the peptide linkers comprise a combination of (G₄X)ₙ, (X'P)ₘ and G_{q}, wherein n, m, q, X, X' have the meanings described in the present invention.

In some embodiments, the first peptide linker and/or the second peptide linker comprise (G₄X)ₙ₁-G_{q1}-(X'P)ₘ-(G₄X)ₙ₂-G_{q2}, wherein each of n1, q1, m, n2, q2 is independently selected from an integer of 1-10, and X is S or A, X' is A, K or E.

In some embodiments, the first peptide linker and/or the second peptide linker comprise (G₄X)ₙ₁-G_{q1}-(X'P)ₘ-(G₄X)ₙ₂-G_{q2}, wherein n1 is 1, q1 is 4, m is 10, n2 is 1, q2 is 4.

In some embodiments, each of the first peptide linker and the second peptide linker independently comprises an amino acid sequence selected from the group consisting of:
GGGGSGGGGSGGGGS (SEQ ID NO: 78), GGGGAPAPAPAPAPAPAPAPAPAPGGGG (SEQ ID NO: 79), GGGGSGGGGSAPAPAPAPAPAPAPAPAPAPGGGGSGGGGS (SEQ ID NO: 80), GGGGSGGGGSEPEPEPEPEPEPEPEPEPEPGGGGSGGGGS (SEQ ID NO: 81).

In some embodiments, the fusion protein comprises an amino acid sequence selected from one of the followings: SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77.

In another embodiment, the fusion protein comprises an amino acid sequence having at least 85%, 90%, 95% or 99% sequence identity to one of the following sequences: SEQ ID NO: 59-77.

**Table 4 GLP-1-Fc-GDF15 fusion protein**

| SEQ ID NO: | Name | GLP-1 polypeptide | Immunoglobulin Fc region | GDF15 active domain |
|---|---|---|---|---|
| 59 | D1 | SEQ ID NO: 2 GLP-1(7-37) | SEQ ID NO: 12 Fc (APEAA) | SEQ ID NO: 38 |
| | | | | WT |
| 60 | D2 | SEQ ID NO: 2 GLP-1(7-37) | SEQ ID NO: 16 Fc (APEAA, K439E) | SEQ ID NO: 38 |
| | | | | WT |
| 61 | D3 | SEQ ID NO: 2 GLP-1(7-37) | SEQ ID NO: 30 Fc (APEAA, K439Q) | SEQ ID NO: 38 |
| | | | | WT |
| 62 | D4 | SEQ ID NO: 2 GLP-1(7-37) | SEQ ID NO: 11 Fc (AA) | SEQ ID NO: 38 |
| | | | | WT |
| 63 | D5 | SEQ ID NO: 2 GLP-1(7-37) | SEQ ID NO: 15 Fc (AA, K439E) | SEQ ID NO: 38 |
| | | | | WT |
| 64 | D6 | SEQ ID NO: 2 GLP-1(7-37) | SEQ ID NO: 29 Fc (AA, K439Q) | SEQ ID NO: 38 |
| | | | | WT |
| 65 | D7 | SEQ ID NO: 5 GLP-1(7-37, A8G, G22E, R36G) | SEQ ID NO: 12 Fc (APEAA) | SEQ ID NO: 40 |
| | | | | ΔN4 |
| 66 | D8 | SEQ ID NO: 5 GLP-1(7-37, A8G, G22E, R36G) | SEQ ID NO: 16 Fc (APEAA, K439E) | SEQ ID NO: 40 |
| | | | | ΔN4 |
| 67 | D9 | SEQ ID NO: 5 GLP-1(7-37, A8G, G22E, R36G) | SEQ ID NO: 30 Fc (APEAA, K439Q) | SEQ ID NO: 40 |
| | | | | ΔN4 |
| 68 | D10 | SEQ ID NO: 5 GLP-1(7-37, A8G, G22E, R36G) | SEQ ID NO: 11 Fc (AA) | SEQ ID NO: 40 |
| | | | | ΔN4 |
| 69 | D11 | SEQ ID NO: 5 GLP-1(7-37, A8G, G22E, R36G) | SEQ ID NO: 15 Fc (AA, K439E) | SEQ ID NO: 40 |
| | | | | ΔN4 |
| 70 | D12 | SEQ ID NO: 5 GLP-1(7-37, A8G, G22E, R36G) | SEQ ID NO: 29 Fc (AA, K439Q) | SEQ ID NO: 40 |
| | | | | ΔN4 |
| 71 | D13 | SEQ ID NO: 7 GLP-1(7-37, A8G, G22E, A30Q, R36G) | SEQ ID NO: 15 Fc (AA, K439E) | SEQ ID NO: 40 |
| | | | | ΔN4 |
| 72 | D14 | SEQ ID NO: 8 GLP-1(7-37, A8G, G22E, I29V, A30Q, R36G) | SEQ ID NO: 15 Fc (AA, K439E) | SEQ ID NO: 40 |
| | | | | ΔN4 |
| 73 | D15 | SEQ ID NO: 5 GLP-1(7-37, A8G, G22E, R36G) | SEQ ID NO: 36 Fc (Hinge, AA, K439E) | SEQ ID NO: 40 |
| | | | | ΔN4 |
| 74 | D16 | SEQ ID NO: 5 GLP-1(7-37, A8G, G22E, R36G) | SEQ ID NO: 37 (APEAA, K439E) | SEQ ID NO: 40 |
| | | | | ΔN4 |
| 75 | D17 | SEQ ID NO: 7 GLP-1(7-37, A8G, G22E, A30Q, R36G) | SEQ ID NO: 37 (APEAA, K439E) | SEQ ID NO: 40 |
| | | | | ΔN4 |
| 76 | D18 | SEQ ID NO: 8 GLP-1(7-37, A8G, G22E, I29V, A30Q, R36G) | SEQ ID NO: 37 (APEAA, K439E) | SEQ ID NO: 40 |
| | | | | ΔN4 |
| 77 | D19 | SEQ ID NO: 5 GLP-1(7-37, A8G, G22E, R36G) | SEQ ID NO: 37 (APEAA, K439E) | SEQ ID NO: 40 |
| | | | | ΔN4 |

| | | | | |
|---|---|---|---|---|
| Note: In Table 4, the numbering method for indicating amino acid positions in GLP-1 polypeptide is as follows: the first amino acid at the N-terminus of SEQ ID NO: 1 is the first position, and the numbers are sequentially numbered in the direction of the C-terminus; the numbering of the amino acid positions in immunoglobulin Fc region follows the EU numbering; the numbering method for indicating amino acid positions in the GDF15 active domain is as follows: the first amino acid at the N-terminus of SEQ ID NO: 38 is the first position, and the numbers are sequentially numbered in the direction of the C-terminus. | | | | |

In the second aspect, the present invention provides a homodimeric fusion protein comprising the fusion protein described in the first aspect of the present invention.

In the third aspect, the present invention provides a biological material, wherein the biological material is any one of (i), (ii), and (iii):
(i) a nucleic acid comprising a nucleotide sequence encoding the fusion protein of the present invention;
(ii) a vector comprising the nucleic acid of (i);
(iii) a host cell comprising the nucleic acid of (i) and/or the vector of (ii).

In the fourth aspect, the present invention provides a method for preparing the fusion protein comprising culturing the host cell of the third aspect of the present invention under conditions suitable for expressing the fusion protein, and purifying the fusion protein expressed by the host cell.

In the fifth aspect, the present invention provides a pharmaceutical composition comprising the fusion protein of the first aspect and/or the homodimeric fusion protein of the second aspect of the present invention as an active ingredient, and the fusion protein and/or the homodimeric fusion protein are present in the pharmaceutical composition in a therapeutically effective amount.

Further, the pharmaceutical composition also comprises a pharmaceutically acceptable carrier.

In the sixth aspect, the present invention provides use of the fusion protein, the homodimeric fusion protein, or the pharmaceutical composition in the manufacture of a medicament for treating metabolic diseases.

Further, the metabolic diseases comprise type II diabetes, obesity, dyslipidemia, diabetic nephropathy, non-alcoholic steatohepatitis, non-alcoholic fatty liver disease, etc.

In the seventh aspect, the present invention provides use of the fusion protein, homodimeric fusion protein, or the pharmaceutical composition in the manufacture of a medicament for reducing food intake, body weight, insulin level, triglyceride level, cholesterol level or glucose level in a subject.

In the eighth aspect, the present invention provides a method for treating metabolic diseases, wherein the method for treating metabolic diseases comprising administering the fusion protein, homodimeric fusion protein, or pharmaceutical composition of the present invention to a subject.

Further, the metabolic disease has the meaning described in the present invention.

Compared with the prior art, the present invention has the following advancements:

The present invention constructs a long-acting GLP-1/GDF15 dual-target fusion protein, and through mutation transformation, the fusion protein has significantly improved physicochemical properties and stability. In the GLP-1-Fc-GDF15 fusion protein provided herein, the two target molecules of GLP-1 and GDF15 have more balanced biological activities in vivo and in vitro, which are more conducive to the exertion of the dual-target effect, and have the superposition or synergistic effect of weight loss of the two targets and other effects of improving metabolic abnormalities.

### DESCRIPTION OF THE DRAWINGS

Various other advantages and benefits will become apparent to those of ordinary skill in the art upon reading the following detailed description of the preferred embodiments. The drawings are for the purpose of illustrating preferred embodiments only and are not to be considered limiting of the invention. In the drawings:
Figure 1: Schematic diagram of Fc-GDF15 fusion protein;
Figure 2: Schematic diagram of the GLP-1-Fc-GDF15 fusion protein provided by the invention;
Figure 3: Effect of repeated administration of GLP-1-Fc-GDF15 fusion protein for 14 days on the body weight of high-fat fed ob/ob mice;
Figure 4: Effect of repeated administration of GLP-1-Fc-GDF15 fusion protein for 14 days on food intake of high-fat fed ob/ob mice;
Figure 5: Effect of repeated administration of GLP-1-Fc-GDF15 fusion protein for 28 days on the body weight of ob/ob mice;
Figure 6: Effect of repeated administration of GLP-1-Fc-GDF15 fusion protein for 28 days on food intake of ob/ob mice;
Figure 7: Effect of repeated administration of GLP-1-Fc-GDF15 fusion protein on the body weight of DIO mice;
Figure 8: Effect of repeated administration of GLP-1-Fc-GDF15 fusion protein on food intake of DIO mice.

### EXAMPLES

Exemplary embodiments of the present disclosure will be described in more detail below with reference to the accompanying drawings. While exemplary embodiments of the present disclosure are shown in the drawings, it should be understood that the present disclosure may be embodied in various forms and should not be limited by the embodiments set forth herein. Rather, these embodiments are provided so that the present disclosure will be more thoroughly understood, and will fully convey the scope of the present disclosure to those skilled in the art.

Unless otherwise defined, all technical and scientific terms used in the present invention have the same meanings as commonly understood by one of ordinary skill in the art. In addition to the specific methods, devices and materials used in the embodiments, according to the mastery of the prior art by those skilled in the art and the description of the present invention, any methods, devices and materials in the prior art that are similar or equivalent to the methods, devices and materials described in the embodiments of the present invention can also be used to implement the present invention.

As used herein, the terms "native", "wild-type" or "WT" refer to a protein or polypeptide that does not contain any genetically engineered mutation, that is naturally occurring or that can be isolated from the environment.

As used herein, the term "basic biological activity" refers to the ability of a polypeptide to retain at least part (for example, not less than about 20%, 30%, 40%, 50%, 60%, 70%, 80% or 90%) or all of its pre-mutation biological activity after mutation.

As used herein, an amino acid "substitution" or "mutation" refers to the replacement of one amino acid in a polypeptide with another amino acid.

As used herein, the amino acid substitution is indicated by a first letter followed by a number and followed by a second letter. The first letter refers to the amino acid in the wild-type protein; the number refers to the amino acid position in which it was substituted; the second letter refers to the amino acid used to replace the wild-type amino acid.

As used herein, amino-terminus deletions of proteins or polypeptides are indicated by ΔN followed by a number, wherein the number represents the number of amino-terminusly deleted amino acids.

The glucagon-like peptide-1 receptors (GLP-1R) are G protein-coupled receptors (GPCRs) composed of 463 amino acids. GLP - 1R is regulated by endogenous GLP-1 polypeptides including GLP-1(1-37), GLP-1(7-37), GLP-1(1-36) and GLP-1(7-36) and exogenous polypeptide exendin-4. In some embodiments, in the fusion protein provided herein, the GLP-1 polypeptide can be selected from GLP-1(1-37) (the amino acid sequence is SEQ ID NO: 1), GLP-1(7-37) (the amino acid sequence is SEQ ID NO: 2), GLP-1(1-36) (the amino acid sequence is SEQ ID NO: 3), and GLP-1(7-36) (SEQ ID NO: 4); or, in other embodiments, in the fusion protein, the GLP-1 polypeptide may have one of the following mutations A8G, G22E, I29V, A30E, A30Q, R36G, or any two or more combinations of different positions (the position of the mutation point follows the numbering in GLP-1(1-37)), for example, GLP-1 with mutations can be GLP-1(7-37, A8G, G22E, R36G) (the amino acid sequence is SEQ ID NO: 5), GLP-1(7-37, A8G, G22E, A30E, R36G) (the amino acid sequence is SEQ ID NO: 6), GLP-1(7-37, A8G, G22E, A30Q, R36G) (the amino acid sequence is SEQ ID NO: 7), GLP-1(7-37, A8G, G22E, I29V, A30Q, R36G) (the amino acid sequence is SEQ ID NO: 8).

As used herein, "growth differentiation factor 15", "GDF15" or "GDF15 active domain" is native mature human GDF15 or a domain thereof in some embodiments, and in other embodiments refers to a mutant of GDF15 or a domain thereof. Mature GDF15 consists of amino acids 197 (A) to 308 (I) of a total of 308 amino acids (UniProt Q99988) excluding the signal peptide and propeptide (GDF15 (197-308) (SEQ ID NO: 46)), or a polypeptide having at least 85%, 90%, 95%, 99% sequence identity to the amino acid sequence within the scope of maintaining the unique activity and structure of GDF15. The mutant comprises truncations, and/or one or more amino acid mutations. In some embodiments, the truncated GDF15 can be an N-terminus deletion variant, such as a sequence having 1-14 amino acid truncations at N-terminus, or a polypeptide having at least 85%, 90%, 95%, 99% sequence identity to the truncated amino acid sequence; in some embodiments, the GDF15 variant refers to a substitution, insertion or deletion of at least one amino acid position compared to mature GDF15 or truncated GDF15, as long as it maintains at least one biological activities of GDF15 protein, such as its effect on food intake, blood glucose levels, insulin resistance, body weight, etc., they are all within the scope of the present invention.

The GDF15 variants can also be produced by introducing one or more conservative or non-conservative amino acid substitutions at specific positions of the GDF15 polypeptide and using naturally occurring or non-naturally occurring amino acids, or by deleting specific residues or segments of residues.

"Conservative amino acid substitutions" may involve a natural amino acid residue (i.e., a residue found at a given position in the wild-type GDF15 polypeptide sequence) being substituted by a non-natural residue (i.e., not a residue found at a given position in the wild-type GDF15 polypeptide sequence) such that there is little or no effect on the polarity or charge of the amino acid residue at that position. Conservative amino acid substitutions also encompass non-naturally occurring amino acid residues (as defined herein) that are typically incorporated by chemical peptide synthesis rather than by synthesis in a biological system. These include peptidomimetics as well as other reversed or inverted forms of amino acid moieties.

The naturally occurring amino acid residues can be grouped into the following classes based on common side chain properties:
(1) Hydrophobic residues: Met, Ala, Val, Leu, Ile;
(2) Neutral hydrophilic residues: Cys, Ser, Thr, Asn, Gln;
(3) Acid residues: Asp, Glu;
(4) Alkaline residues: His, Lys, Arg;
(5) Residues affecting chain orientation: Gly, Pro; and
(6) Aromatic residues: Trp, Tyr, Phe.

Some conservative amino acid substitutions are shown in the table below:

| Amino acids | Conservative amino acid substitutions |
|---|---|
| Ala | D-Ala, Gly, Aib, β-Ala, L-Cys, D-Cys |
| Arg | D-Arg, Lys, Orn, D-Om |
| Asn | D-Asn, Asp, D-Asp, Glu, D-Glu, Gln, D-Gln |
| Asp | D-Asp, D-Asn, Asn, Glu, D-Glu, Gln, D-Gln |
| Cys | D-Cys, S-Me-Cys, Met, D-Met, Thr, D-Thr, L-Ser, D-Ser |
| Gln | D-Gln, Asn, D-Asn, Glu, D-Glu, Asp, D-Asp |
| Glu | D-Glu, Asp, D-Asp, Asn, D-Asn, Gln, D-Gln |
| Gly | Ala, D-Ala, Pro, D-Pro, Aib, β-Ala |
| Ile | D-Ile, Val, D-Val, Leu, D-Leu, Met, D-Met |
| Leu | D-Leu, Val, D-Val, Met, D-Met, Ile, D-Ile |
| Lys | D-Lys, Arg, D-Arg, Orn, D-Om |
| Met | D-Met, S-Me-Cys, Ile, D-Ile, Leu, D-Leu, Val, D-Val |
| Phe | D-Phe, Tyr, D-Tyr, His, D-His, Trp, D-Trp |
| Pro | D-Pro |
| Ser | D-Ser, Thr, D-Thr, allo-Thr, L-Cys, D-Cys |
| Thr | D-Thr, Ser, D-Ser, allo-Thr, Met, D-Met, Val, D-Val |
| Tyr | D-Tyr, Phe, D-Phe, His, D-His, Tip, D-Trp |
| Val | D-Val, Leu, D-Leu, Ile, D-Ile, Met, D-Met |

A conservative substitution may involve the exchange of a member of one of these categories with another member of the same category. Non-conservative substitutions may involve the exchange of members of one of these categories with members of another category.

As used herein, "identity" or "homology" generally refers to the sequence similarity between two peptides or proteins or between two nucleic acid molecules. Percent "identity" or "homology" refers to the percentage of identical residues between amino acids or nucleotides in the molecules being compared, and is calculated based on the size of the smallest molecule in the molecules being compared.

The fusion protein formed by the Fc variant of the present invention and the natural mature human GDF15 or its variant can achieve the purpose of the present invention.

As used herein, "Fc" or "Fc region" is used to define the C-terminus region of the heavy chain of an antibody that contains at least a portion of the constant region. The Fc region can be a native sequence Fc region or a variant Fc region. Those skilled in the art know that an Fc region generally comprises two constant domains: CH₂ and CH₃, and that the boundaries of the Fc region can vary. As used herein, the C-terminus of the Fc region is the C-terminus of the heavy chain of the antibody; while the N-terminus of the Fc region may vary. In some embodiments, the N-terminus of the Fc region may start from, for example, position 231 (EU numbering), or position 233 (EU numbering), or position 236 (EU numbering), or position 237 (EU numbering); in other embodiments, the N-terminus of the Fc region of the present invention does not comprise a hinge region; in yet other embodiments, the N-terminus of the Fc region of the present invention comprises a hinge region. As used herein, the numbering of amino acid residues in the Fc region is according to the EU numbering system, also known as the EU index, as described in Edelman et al., The covalent structure of an entire γG immunoglobulin molecule. Proc. Natl. Acad. Sci., USA, 1969.

In the fusion protein provided herein, natural immunoglobulin Fc region or immunoglobulin Fc region variant is used, in a preferred embodiment, Fc region variant is used. GDF15 needs to form a dimer to exercise its activity. During the research process of the present invention, it was found that although IgG Fc can naturally form a dimer, the spatial distance between the carboxyl terminus of the two monomer Fc in the IgG Fc dimer is significantly different from that of the amino terminus of the GDF15 monomer; even if a flexible or rigid peptide linker is used, the fusion protein formed by the C-terminus of IgG Fc and the N-terminus of GDF15 still has issues such as instability and low expression yield during assembly. The Fc variants provided by the present invention significantly improve the stability of the Fc-GDF15 molecule, realizes the efficient assembly of the Fc-GDF15 fusion protein, and increases the expression yield. The manufacturing process is simple, and the fusion proteins retain the ability to form homodimers. In a preferred embodiment of the present invention, a GLP-1-Fc-GDF15 fusion protein is obtained based on the Fc region variant, so that the fusion protein has significantly improved physicochemical properties and stability compared to the fusion protein using the natural Fc region, and wherein the two target molecules have a better balance of activity and pharmacokinetic characteristics, which is more conducive to the dual-target effect.

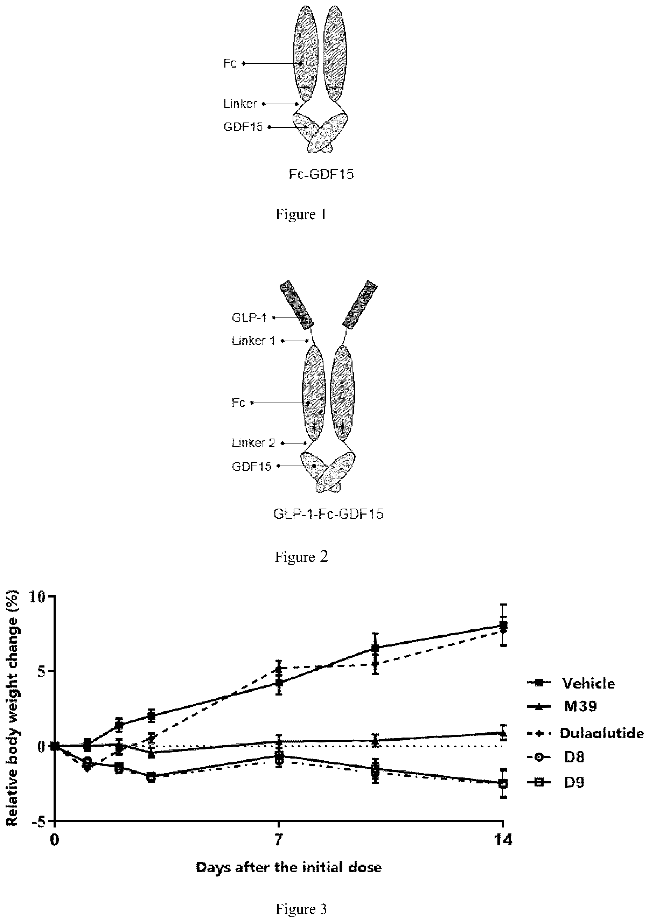

As used herein, "dimer" refers to a protein dimer, which is composed of two protein (polypeptide) molecules, and is the quaternary structure of a protein. The two protein (polypeptide) molecules that make up the dimer may be referred to as the monomer molecule or monomer protein of the dimer. Dimers can include both "homodimers" and "heterodimers", wherein a homodimer is formed by the combination of two identical monomer molecules; a heterodimer is formed by the combination of two different monomer molecules. It is generally believed that homodimers are simpler to prepare than heterodimers because only one peptide needs to be encoded. In the present invention, the Fc variant provided by the present invention has the ability to form homodimers; the fusion protein of the Fc variant provided by the present invention and the GDF15 active domain also has the ability to form homodimers. Referring to Figure 1, in the homodimer formed by the fusion protein, homodimerization exists between two monomer Fc variants and between two monomer GDF15 active domains, respectively. On this basis, the fusion protein provided by the present invention including GLP-1, Fc region variants and GDF15 can also form homodimers, referring to Figure 2.

As used herein, a "linker peptide" or "peptide linker" refers to a single amino acid or polypeptide sequence that joins two proteins together. The length of peptide linkers can be, for example, about 1-40 amino acids, including, for example, repeated alanines, glycines, and serines. Common peptide linkers include flexible peptide linkers, for example, a combination of glycine and serine, such as (GGGGS)ₙ, which can adjust the distance between the connected proteins by the number of repeat units in the peptide linker; peptide linkers composed only of glycine, such as Gₙ, common ones include G₆, Gs, *etc.;* rigid peptide linkers, such as α-helical peptide linkers with the (EAAAK)ₙ sequence; peptide linkers with proline-rich (XP)ₙ, wherein X can specify any amino acid, common ones include alanine, lysine, or glutamic acid, (XP)ₙ sequence has no helical structure, and the presence of proline in the peptide linker can increase backbone stiffness and effectively separate domains. Structures with proline-rich sequences are widespread in the body, such as the structure of (AP)₇ at the N-terminus of skeletal muscle proteins. The selection of peptide linkers can be determined by those skilled in the art based on usual experiments, for example, using only flexible peptide linkers, using only rigid peptide linkers, using a combination of flexible peptide linkers and rigid peptide linkers, and the like.

As used herein, "vector" refers to a nucleic acid molecule capable of propagating another nucleic acid to which it is linked, including vectors that are self-replicating nucleic acid structures and vectors that integrate into the genome of a host cell into which it is introduced. Certain vectors are capable of directing the expression of nucleic acids to which they are operably linked, and such vectors are referred to herein as "expression vectors".

As used herein, "host cell" refers to a cell into which exogenous nucleic acid has been introduced, including progeny of such cells. Host cells include the initially transformed cell and progeny derived therefrom (regardless of passage number). The progeny may not be identical to the parent cell in the content of nucleic acid, but may contain mutations. Mutant progeny having the same function or biological activity as screened or selected in the original transformed cell are included herein. Host cells are any type of cellular system that can be used to produce the fusion proteins of the present invention. Host cells include mammalian cultured cells such as CHO cells, BHK cells, NS0 cells, SP2/0 cells, YO myeloma cells, P3X63 mouse myeloma cells, PER cells, PER.C6 cells or hybridoma cells, yeast cells, bacterial cells such as E. coli, insect cells and plant cells, etc., and also include cells contained in transgenic animals, transgenic plants, or cultured plant or animal tissue.

As used herein, a "pharmaceutical composition" refers to a formulation in a form that permits the biological activity of the active ingredient contained therein to be effective and free of additional ingredients that would be unacceptably toxic to subjects to whom the pharmaceutical composition would be administered.

As used herein, a "therapeutically effective amount" refers to an amount effective to achieve a desired therapeutic or prophylactic result, including, for example, eliminating, reducing, delaying, minimizing or preventing adverse effects of a disease.

As used herein, a "pharmaceutically acceptable carrier" refers to ingredients other than the active ingredient in the pharmaceutical composition that are not toxic to the subject. Pharmaceutically acceptable carriers include, but are not limited to, buffers, excipients, stabilizers or preservatives.

As used herein, "treatment" refers to an attempt to alter the natural course of disease in a treated individual, and may be for prevention or clinical intervention performed during the course of clinical pathology. Desired effects of treatment include, but are not limited to, preventing the occurrence or recurrence of the disease, alleviating symptoms, reducing any direct or indirect pathological consequences of the disease, slowing the rate of disease progression, improving or eliminating the disease state, and regressing or improving the prognosis.

As used herein, an "individual" or a "subject" is a mammal. Mammals include, but are not limited to, primates (e.g., humans and non-human primates such as monkeys) or other mammals (e.g., cows, sheep, cats, dogs, horses, rabbits, and rodents such as mice and rats). In particular, the individual or subject is a human.

As used herein, the conventional one-letter or three-letter codes for natural amino acids are used:

| | |
|---|---|
| Alanine (Ala, A) | Arginine (Arg, R) |
| Asparagine (Asn, N) | Aspartic acid (Asp, D) |
| Cysteine (Cys, C) | Glutamic acid (Glu, E) |
| Glutamine (Gln, Q) | Glycine (Gly, G) |
| Histidine (His, H) | Isoleucine (Ile, I) |
| Leucine (Leu, L) | Lysine (Lys, K) |
| Methionine (Met, M) | Phenylalanine (Phe, F) |
| Proline (Pro, P) | Serine (Ser, S) |
| Threonine (Thr, T) | Tryptophan (Trp, W) |
| Tyrosine (Tyr, Y) | Valine (Val, V) |

### Example 1 Design of Fc variant

In this example, the effects of introducing different mutations on the structural properties of Fc were tested, and the test results of some mutants are shown in Table 5. Test steps include:

Molecular cloning method was used to insert Fc nucleic acid sequence into mammalian cell expression vector, and ExpiCHO Fectamine^{™} CHO Transfection Kit (ThermoFisher Scientific) was used for transient transfection and expression in CHO-S cells; after purification by Protein A column, the target protein was obtained, and the target protein was analyzed by size exclusion high performance liquid chromatography (SEC). The main principle of SEC analysis is based on the molecular weight and three-dimensional structure of the analyte (such as protein). Generally, analytes with larger molecular weights will pass through the chromatographic column more quickly, so they have a shorter column retention time. On the contrary, analytes with smaller molecular weights have longer retention times. So SEC can be used to analyze the aggregation of protein molecules.

The natural immunoglobulin Fc fragment will naturally form a homodimer structure. In order to improve the stability of the fusion protein, the Fc molecule needs to be mutated, which may destroy the structure of Fc homodimer. However, the inventors surprisingly found that the Fc molecule (K439D or K439E) after the introduction of the mutation at position 439 had the same retention time in the SEC analysis as the Fc molecule without mutation, indicating that the introduced mutation did not disrupt the formation of Fc dimer. As a control, according to a mutant molecule (F405Q, Y407E) that disrupts the formation of Fc dimer reported in the previous patent document WO2019195091, this example also tested the Fc molecule with the amino acid sequence of SEQ ID NO: 82, and found that it has a significantly extended retention time, indicating that the Fc molecule exists in monomer form.

**Table 5 Properties of the formation of Fc variant dimer**

| Sequences | Fc variant | Retention time | Aggregate state |
|---|---|---|---|
| SEQ ID NO: 11 | IgG4 Fc (AA) | 13.341 min | Dimer |
| SEQ ID NO: 15 | IgG4 Fc (AA, K439E) | 13.156 min | Dimer |
| SEQ ID NO: 19 | IgG4 Fc (AA, K439D) | 13.093 min | Dimer |
| SEQ ID NO: 82 | IgG4 Fc (AA, F405Q/Y407E) | 15.076 min | Monomer |

### Example 2 Preparation of Fc-GDF15 construct

Construction of expression vectors: The gene sequence encoding the Fc-GDF15 fusion protein shown in Table 6 was inserted into the polyclonal enzyme cleavage site of mammalian cell vector pXC17.4 to construct the expression vectors expressing Fc-GDF15 as shown in Table 6, and the expression vectors of Fc-GDF15 were extracted by endotoxin-free plasmid extraction kit (OMEGA) for cell transfection.

**Table 6 Fc-GDF15 fusion protein**

| SEQ ID NO: | Name | Fc variant sequenc e SEQ ID NO: | Note of mutation in Fc variant | GDF 15 active domain sequence SEQ ID NO: | Note of GDF active domain |
|---|---|---|---|---|---|
| 83 | M1 | 12 | Fc (APEAA) | 40 | ΔN4 |
| 84 | M2 | 16 | Fc (APEAA, K439E) | 38 | WT |
| 85 | M3 | 16 | Fc (APEAA, K439E) | 39 | ΔN3 |
| 86 | M4 | 16 | Fc (APEAA, K439E) | 40 | ΔN4 |
| 87 | M5 | 20 | Fc (APEAA, K439D) | 40 | ΔN4 |
| 88 | M6 | 30 | Fc (APEAA, K439Q) | 40 | ΔN4 |
| 89 | M7 | 32 | Fc (APEAA, K439A) | 40 | ΔN4 |
| 90 | M8 | 24 | Fc (APEAA, E356K) | 40 | ΔN4 |
| 91 | M9 | 28 | Fc (APEAA, E356R) | 40 | ΔN4 |
| 92 | M10 | 33 | Fc (APEAA, E356Q) | 40 | ΔN4 |
| 93 | M11 | 35 | Fc (APEAA, E356A) | 40 | ΔN4 |
| 94 | M12 | 37 | IgG1 Fc (APEAA, K439E) | 40 | ΔN4 |
| 95 | M13 | 15 | Fc (AA, K439E) | 40 | ΔN4 |
| 96 | M14 | 10 | Fc (GG) | 40 | ΔN4 |
| 97 | M15 | 14 | Fc (GG, K439E) | 40 | ΔN4 |
| 98 | M16 | 13 | Fc (G, K439E) | 40 | ΔN4 |
| 99 | M17 | 14 | Fc (GG, K439E) | 40 | ΔN4 |
| 100 | M18 | 14 | Fc (GG, K439E) | 40 | ΔN4 |
| 101 | M19 | 14 | Fc (GG, K439E) | 40 | ΔN4 |
| 102 | M20 | 16 | Fc (APEAA, K439E) | 42 | ΔN4, D5E |
| 103 | M21 | 16 | Fc (APEAA, K439E) | 43 | ΔN5, H6D |
| 104 | M22 | 16 | Fc (APEAA, K439E) | 44 | ΔN4, H6E |
| 105 | M23 | 16 | Fc (APEAA, K439E) | 45 | ΔN5, H6E |
| 106 | M24 | 16 | Fc (APEAA, K439E) | 46 | ΔN4, R21Q |
| 107 | M25 | 16 | Fc (APEAA, K439E) | 47 | ΔN4, R21H |
| 108 | M26 | 16 | Fc (APEAA, K439E) | 48 | ΔN4, D26E |
| 109 | M27 | 16 | Fc (APEAA, K439E) | 49 | ΔN4, D26E, K69R |
| 110 | M28 | 16 | Fc (APEAA, K439E) | 50 | ΔN4, A30S |
| 111 | M29 | 16 | Fc (APEAA, K439E) | 51 | ΔN4, A47D |
| 112 | M30 | 16 | Fc (APEAA, K439E) | 52 | ΔN4, A54S |
| 113 | M31 | 16 | Fc (APEAA, K439E) | 53 | ΔN4, A55E |
| 114 | M32 | 16 | Fc (APEAA, K439E) | 54 | ΔN4, M57T |
| 115 | M33 | 16 | Fc (APEAA, K439E) | 55 | ΔN4, R67Q |
| 116 | M34 | 16 | Fc (APEAA, K439E) | 56 | ΔN4, A81S |
| 117 | M3 5 | 16 | Fc (APEAA, K439E) | 57 | ΔN4, T94E |
| 118 | M3 6 | 16 | Fc (APEAA, K439E) | 58 | ΔN4, K107Q |
| 119 | M3 7 | 16 | Fc (APEAA, K439E) | 41 | ΔN14 |
| 120 | M3 9 | 30 | Fc (APEAA, K439Q) | 39 | ΔN3 |
| 121 | M40 | 33 | Fc (APEAA, E356Q) | 39 | ΔN3 |
| 122 | M41 | 31 | Fc (APEAA, K439N) | 39 | ΔN3 |
| 123 | M42 | 34 | Fc (APEAA, E356N) | 39 | ΔN3 |
| 124 | M43 | 32 | Fc (APEAA, K439A) | 39 | ΔN3 |
| 125 | M44 | 35 | Fc (APEAA, E356A) | 39 | ΔN3 |
| 126 | M45 | 30 | Fc (APEAA, K439Q) | 39 | ΔN3 |
| 127 | M46 | 16 | Fc (APEAA, K439E) | 39 | ΔN3 |
| 128 | M47 | 20 | Fc (APEAA, K439D) | 39 | ΔN3 |
| 129 | M48 | 24 | Fc (APEAA, E356K) | 39 | ΔN3 |
| 130 | M49 | 28 | Fc (APEAA, E356R) | 39 | ΔN3 |
| 131 | M50 | / | Fc (APEAA, E356Q, K439Q) | 39 | ΔN3 |
| 132 | M51 | / | Fc (APEAA, K439N) | 40 | ΔN4 |
| 133 | M52 | / | Fc (APEAA, E356N) | 40 | ΔN4 |
| 134 | M53 | / | Fc (GG, K439E) | 40 | ΔN4 |
| 135 | M54 | / | Fc (APEAA, E356D) | 40 | ΔN4 |
| 136 | M55 | / | Fc (APEAA, K439R) | 40 | ΔN4 |

Cell transfection and culture: CHO-S cells were recovered and subcultured, and the cells were collected when the density was about 6×10⁶ cells/mL for cell transfection. Cells were transfected using ExpiCHO Fectamine^{™} CHO Transfection Kit (ThermoFisher Scientific), in which the final concentration of Fc-GDF15 expression vector was 1 µg/mL. About 20 hours after transfection, ExpiCHO Fectamine CHO Enhancer and ExpiCHO Feed were added to maintain the growth of transfected cells. The cell culture medium was harvested when the cell viability decreased to about 80%.

Protein purification: After centrifuging the cell culture medium, the supernatant was collected and filtered with a 0.22 µm filter to obtain the cell culture supernatant. The cell culture supernatant was purified using a two-step chromatography method. The first step of chromatography: The cell culture supernatant was applied to an AT Protein A Diamond column (Borgron Corporation) equilibrated with phosphate buffered saline (PBS). After the target protein was bound, the mixture was washed with equilibration solution for more than 3CV (column volume) to remove unbound impurities, and eluted with 100mM acetic acid-sodium acetate (pH 3.0) buffer. The target protein was collected, then the pH of the collected sample was quickly adjusted to 7.2-7.6 with 1.0M Tris-HCl pH 8.0 solution, and the pH adjusted sample was diluted with purified water until its conductivity value was lower than 5ms/cm to obtain the first sample. The second step of chromatography: The first sample obtained by the first layer chromatography was further purified using a Bestarose Q HP column, which was equilibrated with 20 mM Tris-HCl buffer. The first sample was loaded, washed with 20 mM Tris-HCl buffer for 3CV and 20mM PB buffer for more than 3CV to replace the buffer system, and finally eluted with PBS. The elution fraction was collected to obtain the target protein sample. The nature and purity of target protein samples were evaluated by sodium dodecyl sulfate polyacrylamide gel electrophoresis (SDS-PAGE) and mass spectrometry analysis. The target protein samples were quantified by a micro-nucleic acid protein analyzer (NanoDrop 2000/2000c Spectrophotometer).

### Example 3 Effect of Fc variants on the expression yield of Fc-GDF15 fusion protein

The Fc-GDF15 fusion proteins described in Table 6 were expressed by the method described in Example 2, and the effects of applying different Fc variants on the expression yield of Fc-GDF15 fusion proteins were compared. The results of the tests are shown in Table 7.

According to the test results, it was found that the Fc-GDF15 fusion proteins that did not mutate at K439 (EU numbering) or E356 (EU numbering) of Fc, such as M1 (a homodimer with a monomer having the sequence of SEQ ID NO: 83), M14 (a homodimer with a monomer having the sequence of SEQ ID NO: 96), had lower expression yields, while the Fc-GDF15 fusion proteins mutated at K439 and/or E356 of Fc, such as M2 (a homodimer with a monomer having the sequence of SEQ ID NO: 84), M3 (a homodimer with a monomer having the sequence of SEQ ID NO: 85), M4 (a homodimer with a monomer having the sequence of SEQ ID NO: 86), M5 (a homodimer with a monomer having the sequence of SEQ ID NO: 87), M6 (a homodimer with a monomer with the sequence of SEQ ID NO: 88), M7 (a homodimer with a monomer with the sequence of SEQ ID NO: 89), M8 (a homodimer with a monomer having the sequence of SEQ ID NO: 90), M9 (a homodimer with a monomer having the sequence of SEQ ID NO: 91), M10 (a homodimer with a monomer having the sequence of SEQ ID NO: 92), M11 (a homodimer with a monomer having the sequence of SEQ ID NO: 93), M15 (a homodimer with a monomer having the sequence of SEQ ID NO: 97), M39 (a homodimer with a monomer having the sequence of SEQ ID NO: 120), had improved fusion protein expression yields, indicating that the mutation of amino acid at position 356 (EU numbering) or 439 (EU numbering) is more favorable for the assembled expression of Fc-GDF15 fusion protein.

**Table 7 Expression yield of Fc-GDF15 molecules comprising different Fc variants**

| Fc-GDF15 Construct | Fc mutation at position 356 or 439 | Expression volume (ml) | Expression yield (Protein A purification, mg/mL) |
|---|---|---|---|
| M14 | Unmutated | 50 | 1.7 |
| M15 | K439E | 50 | 12.6 |
| M1 | Unmutated | 100 | 6.7 |
| M2 | K439E | 100 | 14.5 |
| M3 | K439E | 100 | 17.0 |
| M4 | K439E | 100 | 16.9 |
| M5 | K439D | 100 | 13.7 |
| M6 | K439Q | 100 | 29.4 |
| M7 | K439A | 100 | 26.0 |
| M8 | E356K | 100 | 34.5 |
| M9 | E356R | 100 | 27.2 |
| M10 | E356Q | 100 | 15.0 |
| M11 | E356A | 100 | 13.2 |
| M39 | K439Q | 100 | 35.8 |
| M50 | E356Q, K439Q | 100 | 21.5 |

### Example 4 Physicochemical properties of Fc-GDF15 fusion protein

The physicochemical properties of protein molecules, such as their tendency to aggregate and degrade, affect the druggability of the molecule, so an ideal drug molecule needs to have a stable single component, that is, less aggregation and degradation occur.

In order to test the physicochemical properties of the Fc-GDF15 fusion protein, the Fc-GDF15 fusion protein was expressed and purified by the method described in Example 2, and then the fusion protein was analyzed by size exclusion chromatography (SEC). Specifically, a Waters Xbridge BEH 200A, 3.5 µm (7.8 × 300 mm) chromatographic column was used for SEC analysis, the mobile phase was (150 mM phosphate buffer: acetonitrile = 9:1, pH 6.5), and the flow rate was 0.5 ml/min. In the test results, the proportion of high molecular weight (HMW) components represents the proportion of aggregation, and the proportion of low molecular weight (LMW) components represents the proportion of degradation. The test results are shown in Table 8.

**Table 8 Comparison of physicochemical properties of different Fc-GDF15 fusion proteins**

| Construct | Fc mutation at | GDF15 | HMW (%) | LMW (%) |
|---|---|---|---|---|
| | position 356 or 439 | mutation | | |
| M1 | Unmutated | ΔN4 | 50.2 | 0.5 |
| M14 | Unmutated | ΔN4 | 67.6 | 0.7 |
| M2 | K439E | WT | 3.6 | 2.1 |
| M3 | K439E | ΔN3 | 2.4 | 0.1 |
| M4 | K439E | ΔN4 | 2.2 | 0.7 |
| M5 | K439D | ΔN4 | 2.8 | 0.2 |
| M6 | K439Q | ΔN4 | 2.7 | 1.0 |
| M7 | K439A | ΔN4 | 4.4 | 0.9 |
| M8 | E356K | ΔN4 | 7.4 | 0.6 |
| M9 | E356R | ΔN4 | 3.9 | 0.3 |
| M10 | E356Q | ΔN4 | 7.1 | 0.7 |
| M11 | E356A | ΔN4 | 11.2 | 1.7 |
| M13 | K439E | ΔN4 | 2.2 | 0.2 |
| M15 | K439E | ΔN4 | 2.1 | 0.2 |
| M16 | K439E | ΔN4 | 12 | 1.5 |
| M17 | K439E | ΔN4 | 11.0 | 0.3 |
| M18 | K439E | ΔN4 | 0.2 | 0.1 |
| M19 | K439E | ΔN4 | 4.6 | 0.7 |
| M20 | K439E | ΔN4, D5E | 2.2 | 0.1 |
| M21 | K439E | ΔN5, H6D | 2.0 | 1.1 |
| M22 | K439E | ΔN4, H6E | 2.1 | 0.2 |
| M23 | K439E | ΔN5, H6E | 1.8 | 0.2 |
| M24 | K439E | ΔN4, R21Q | 3.1 | 0.1 |
| M25 | K439E | ΔN4, R21H | 1.5 | 0.1 |
| M26 | K439E | ΔN4, D26E | 2.3 | 0.1 |
| M27 | K439E | ΔN4, D26E, K69R | 2.8 | 0.2 |
| M28 | K439E | ΔN4, A30S | 2.3 | 0.1 |
| M29 | K439E | ΔN4, A47D | 1.6 | 0.1 |
| M30 | K439E | ΔN4, A54S | 2.0 | 0.2 |
| M31 | K439E | ΔN4, A55E | 0.7 | 0.1 |
| M32 | K439E | ΔN4, M57T | 2.0 | 0.1 |
| M33 | K439E | ΔN4, R67Q | 1.6 | 0.2 |
| M34 | K439E | ΔN4, A81S | 2.3 | 0.1 |
| M35 | K439E | ΔN4, T94E | 0.7 | 0.1 |
| M36 | K439E | ΔN4, K107Q | 1.2 | 0.1 |
| M37 | K439E | ΔN14 | 1.4 | 1.1 |
| M39 | K439Q | ΔN3 | 3.2 | 0.9 |
| M53 | K439E | ΔN4 | 8.3 | 2.7 |
| M54 | E356D | ΔN4 | 33.7 | 0.7 |
| M55 | K439R | ΔN4 | 36.7 | 0.6 |

The results in Table 8 show that Fc-GDF15 fusion proteins without comprising mutation at K439 (EU numbering) or E356 (EU numbering) of Fc, such as M1 and M14, have 50.2% and 67.6% high molecular weight (HMW) aggregation, respectively; while Fc-GDF15 fusion proteins mutated at K439 (EU numbering) and/or E356 (EU numbering) of Fc all have significantly reduced HMW aggregation ratio, and most fusion proteins have less than 5% HMW aggregation, indicating that the mutation of Fc at K439 (EU numbering) or E356 (EU numbering) can bring better druggability of Fc-GDF15 fusion protein.

### Example 5 Preparation of GLP-1-Fc-GDF15 construct

The GLP-1-Fc-GDF15 fusion protein was constructed by fusing the GLP-1 sequence at the N-terminus of Fc-GDF15. In this example, the GLP-1-Fc-GDF15 fusion protein shown in Table 4 was prepared according to the following steps.

Construction of expression vectors: The gene sequence encoding the GLP-1-Fc-GDF15 fusion protein shown in Table 4 was inserted into the polyclonal enzyme cleavage site of mammalian cell vector pXC17.4 to construct the expression vectors expressing GLP-1-Fc-GDF15 as shown in Table 4, and the expression vectors of GLP-1-Fc-GDF15 were extracted by endotoxin-free plasmid extraction kit (OMEGA) for cell transfection.

Cell transfection and culture: CHO-S cells were recovered and subcultured, and the cells were collected when the density was about 6×10⁶ cells/mL for cell transfection. Cells were transfected using ExpiCHO Fectamine^{™} CHO Transfection Kit (ThermoFisher Scientific), in which the final concentration of GLP-1-Fc-GDF15 expression vector was 1 µg/ml. About 20 hours after transfection, ExpiCHO Fectamine CHO Enhancer and ExpiCHO Feed were added to maintain the growth of transfected cells. The cell culture medium was harvested when the cell viability decreased to about 80%.

Protein purification: After centrifuging the cell culture medium, the supernatant was collected and filtered with a 0.22 µm filter to obtain the cell culture supernatant. The cell culture supernatant was purified using a two-step chromatography method. The first step of chromatography: The cell culture supernatant was applied to an AT Protein A Diamond column (Borgron Corporation) equilibrated with phosphate buffered saline (PBS). After the target protein was bound, the mixture was washed with equilibration solution for more than 3CV (column volume) to remove unbound impurities, and eluted with 100mM acetic acid-sodium acetate (pH 3.0) buffer. The target protein was collected, then the pH of the collected sample was quickly adjusted to 7.2-7.6 with 1.0M Tris-HCl pH 8.0 solution, and the pH adjusted sample was diluted with purified water until its conductivity value was lower than 5ms/cm to obtain the first sample. The second step of chromatography: The first sample obtained by the first layer chromatography was further purified using a Bestarose Q HP column, which was equilibrated with 20 mM Tris-HCl buffer. The first sample was loaded, washed with 20 mM Tris-HCl buffer for 3CV and 20mM PB buffer for more than 3CV to replace the buffer system, and finally eluted with PBS. The elution fraction was collected to obtain the target protein sample. The nature and purity of target protein samples were evaluated by sodium dodecyl sulfate polyacrylamide gel electrophoresis (SDS-PAGE) and mass spectrometry analysis. The target protein samples were quantified by a micro-nucleic acid protein analyzer (NanoDrop 2000/2000c Spectrophotometer).

### Example 6 Physicochemical properties of GLP-1-Fc-GDF15 fusion protein

In order to test the physicochemical properties of the GLP-1-Fc-GDF 15 fusion protein, the GLP-1-Fc-GDF15 fusion protein was expressed and purified by the method described in Example 5, and then the fusion protein was analyzed by size exclusion chromatography (SEC). Specifically, a Waters Xbridge BEH 200A, 3.5 µm (7.8 × 300 mm) chromatographic column was used for SEC analysis, the mobile phase was (150 mM phosphate buffer: acetonitrile = 9:1, pH 6.5), and the flow rate was 0.5 ml/min. In the test results, the proportion of high molecular weight (HMW) components represents the proportion of aggregation, and the proportion of low molecular weight (LMW) components represents the proportion of degradation. The test results are shown in Table 9.

**Table 9 Comparison of physicochemical properties of different GLP-1-Fc-GDF15 fusion proteins**

| Construct | HMW (%) | LMW (%) |
|---|---|---|
| D8 | 0.6 | 1.4 |
| D9 | 0.8 | 2.1 |
| D11 | 0.9 | 0.3 |
| D13 | 0.5 | 1.0 |
| D14 | 3.4 | 1.3 |

The results show that the total aggregation and degradation ratio of the dual-target fusion protein is less than 5%, indicating that the dual-target protein has good physicochemical properties.

### Example 7 In vitro activity of GLP-1-Fc-GDF15 fusion protein

GLP-1 functions by activating its receptor GLP-1R, which binds to the receptor and activates intracellular downstream signaling pathways and produces cAMP, so the in vitro GLP-1 activity of GLP-1-Fc-GDF15 fusion protein can be evaluated by detecting intracellular cAMP levels; GDF15 functions in vivo and requires signal transduction through its specific receptor GFRAL and coreceptor RET. GDF15 binds to cell surface receptors and activates downstream signaling pathways, one of which is ERK1/2 phosphorylation. Therefore, the in vitro activity of GLP-1-Fc-GDF15 fusion protein can be evaluated by measuring the level of ERK1/2 phosphorylation in cells.

HEK293 cell lines with high GLP-1R expression and HEK293T cell lines with high expression of GFRAL and RET were constructed by genetic engineering. The in vitro activities of GLP-1 and GDF15 of GLP-1-Fc-GDF15 fusion protein were evaluated using the cAMP Gs HiRange Kit (cisbio) and the Advanced phospho-ERK1/2 (Thr202/Tyr204) Kit (cisbio), respectively. The EC₅₀ values of in vitro activity were obtained by assay according to the kit manual.

**Table 10 In vitro activity of GLP-1-Fc-GDF15 fusion protein**

| Construct | GLP-1 activity (EC₅₀, nM) | GDF15 activity (EC₅₀, nM) |
|---|---|---|
| D8 | 0.084 | |
| D9 | 0.100 | |
| D11 | 0.118 | |
| D13 | 0.124 | |
| D14 | 0.183 | |

The results show that the GLP-1-Fc-GDF15 fusion protein provided herein retains high in vitro activities of GLP-1 and GDF15 at the same time.

### Example 8 In vivo efficacy of repeated administration of GLP-1-Fc-GDF15 fusion protein in high-fat diet ob/ob (HFD-ob/ob) mice

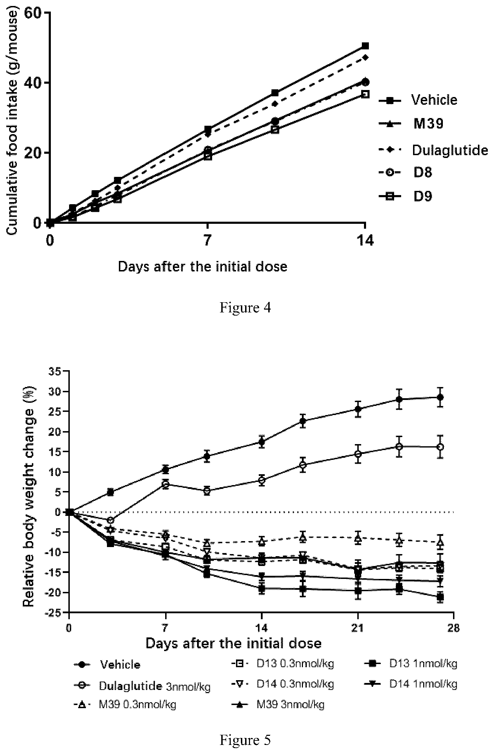

This example tested the effect of repeated administration of GLP-1-Fc-GDF15 fusion protein on appetite suppression and weight reduction in HFD-ob/ob mice. After two weeks of high-fat diet induction, ob/ob mice were randomly divided into groups according to body weight, and then subcutaneously administered with vehicle, GLP-1-Fc-GDF15 fusion proteins D8 and D9 (1 nmol/kg, dosing twice weekly after 7 days of initial dose), GLP-1-Fc fusion protein (Dulaglutide, 1 nmol/kg, dosing twice weekly after 7 days of initial dose), or Fc-GDF15 fusion protein (M39) (1 nmol/kg, dosing once a week), and changes in mouse body weight and food intake were continuously monitored. On the 14th day after the initial dose, the results of mouse body weight and food intake are shown in Table 11, Figure 3 and Figure 4.

**Table 11 Effects of GLP-1-Fc-GDF15 fusion protein treatment on body weight and food intake of HFD-ob/ob mice**

| | Day 14 | |
|---|---|---|
| Group | Relative body weight change (%) | Cumulative food intake (g) |
| Vehicle | 8.07±1.40 | 50.5 |
| M39 | 0.90±0.49***^{###} | 40.7 |
| Dulaglutide | 7.69±0.93 | 47.3 |
| D8 | -2.53±0.13***^{###} | 40.2 |
| D9 | -2.46±0.93***^{###} | 36.8 |

| | | |
|---|---|---|
| Note: Values represent the mean ± SEM of data from 9-10 animals in each group; ***-p<0.001, relative to vehicle; ^{###}-p<0.001, relative to Dulaglutide; statistical analysis method: one-way ANOVA with Turkey's correction. | | |

The results show that GLP-1/GDF15 dual-target molecules D8 and D9 can significantly reduce the food intake of obese mouse models. More importantly, compared with the GLP-1 single-target drugs Dulaglutide and GDF15 single-target molecules M39, dual-target molecules D8 and D9 have more potent weight reduction effects, indicating that the GLP-1-Fc-GDF15 fusion protein designed in the present invention can exert the superposition or synergistic effect of the weight reduction effects of the two targets.

### Example 9 The efficacy of repeated administration of GLP-1-Fc-GDF15 fusion protein in ob/ob mouse model

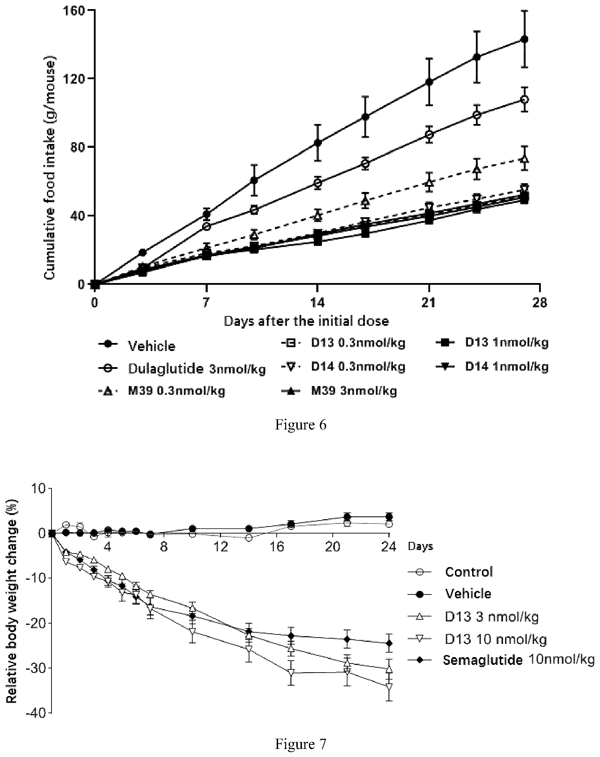

This example tested the effect of repeated administration of GLP-1-Fc-GDF15 fusion protein on reducing food intake, reducing body weight, improving blood biochemical indicators, and improving liver function and liver pathology in ob/ob mice. After the ob/ob mice adapted to the new feeding, they were randomly divided into groups according to body weight, and then subcutaneously administered with vehicle, GLP-1-Fc-GDF15 fusion proteins D13 and D14 (two dose groups: 0.3 nmol/kg and 1 nmol/kg), GLP-1-Fc fusion protein (Dulaglutide, 3nmol/kg) or Fc-GDF15 fusion protein (M39) (two dose groups: 0.3nmol/kg and 3nmol/kg), administered twice weekly after 7 days of initial dose, with a total treatment time of four weeks. Changes of body weight and food intake were continuously monitored. On the 28th day, an autopsy was performed to detect liver pathological indicators, and before the autopsy, blood was collected to detect serum lipids and various biochemical indicators. The results of body weight and food intake are shown in Figure 5, Figure 6 and Table 12, respectively. The improvement results of glycosylated hemoglobin (HbA1c), alanine aminotransferase (ALT), aspartate aminotransferase (AST), and liver pathology (hepatic steatosis) are shown in the Table 13 and Table 14, respectively.

**Table 12 Effects of GLP-1-Fc-GDF15 fusion protein treatment on body weight and food intake of ob/ob mice**

| | Day 27 | |
|---|---|---|
| Group | Relative body weight change (%) | Cumulative food intake (g) |
| Vehicle | 28.61±2.45 | 143.2±16.5 |
| Dulaglutide 3nmol/kg | 16.28±2.77*** | 107.9±7.1 |
| M39 0.3nmol/kg | -7.39±1.82***^{###} | 73.7±6.9 |
| M39 3nmol/kg | -12.55±2.22***^{###} | 52.5±2.9 |
| D13 0.3nmol/kg | -13.96±1.07***^{###} | 51.7±1.2 |
| D13 1nmol/kg | -21.05±1.32***^{###&} | 49.3±0.0 |
| D14 0.3nmol/kg | -13.27±1.12***^{###} | 55.4±3.4 |
| D14 1nmol/kg | -17.17±1.35***^{###} | 50.9±1.8 |

| | | |
|---|---|---|
| Note: Body weight change values represent the mean ± SEM of data from 8 animals in each group; ***-p<0.001, relative to vehicle; ^{###}-p<0.001, relative to Dulaglutide; ^{&}-p<0.05, relative to M39 (3nmol/kg); statistical analysis method: one-way ANOVA with Turkey's correction. | | |

**Table 13 GLP-1-Fc-GDF 15 fusion protein treatment reduces glycosylated hemoglobin levels in ob/ob mice**

| Group | HbAlc (%) |
|---|---|
| Vehicle | 6.07±0.25 |
| Dulaglutide 3nmol/kg | 4.90±0.18*** |
| M39 0.3nmol/kg | 3.91±0.09***^{###} |
| M39 3nmol/kg | 3.73±0.11***^{###} |
| D13 0.3nmol/kg | 3.65±0.08***^{###} |
| D13 1nmol/kg | 3.76±0.14***^{###} |
| D14 0.3nmol/kg | 3.67±0.10***^{###} |
| D14 1nmol/kg | 3.58±0.09***^{###} |

| | |
|---|---|
| Note: Values represent the mean ± SEM of data from 8 animals in each group; ***-p<0.001, relative to vehicle; ^{###}-p<0.001, relative to Dulaglutide; statistical analysis method: one-way ANOVA with Turkey's correction. | |

**Table 14 GLP-1-Fc-GDF 15 fusion protein treatment improves liver function and hepatic steatosis in ob/ob mice**

| Group | ALT (U/L) | AST (U/L) | Steatosis Score |
|---|---|---|---|
| Vehicle | 281.2±25.2 | 337.6±31.0 | 3.00±0.00 |
| Dulaglutide 3nmol/kg | 139.0±16.2* | 219.9±16.6*** | 3.00±0.00*** |
| M39 0.3nmol/kg | 86.3±8.7*** | 164.0±10.5*** | 0.88±0.08***^{###} |
| M39 3nmol/kg | 53.8±2.8*** | 141.8±4.1***^{#} | 0.56±0.15***^{###} |
| D13 0.3nmol/kg | 55.8±1.8*** | 140.9±17.3***^{#} | 0.25±0.09***^{###} |
| D13 1nmol/kg | 66.1±4.0*** | 174.0±16.5 *** | 0.19±0.09***^{###} |
| D14 0.3nmol/kg | 58.0±3.2*** | 143.4±6.1***^{#} | 0.19±0.09***^{###} |
| D14 1nmol/kg | 54.4±3.5*** | 136.5±9.5***^{#} | 0.19±0.13***^{###} |

| | | | |
|---|---|---|---|
| Note: Values represent the mean ± SEM of data from 8 animals in each group; *-p<0.05, ***-p<0.001, relative to vehicle; ^{#}-p<0.05, ^{###}-p<0.001, relative to Dulaglutide; statistical analysis method: one-way ANOVA with Turkey's correction. | | | |

The results show that the GLP-1/GDF15 dual-target fusion proteins D13 and D14 can significantly reduce the food intake and body weight of the ob/ob obesity mouse model, and the effect at the same dose is significantly better than the single-target GLP-1 drug Dulaglutide and GDF15 single-target molecule M39. At the same time, D13 and D14 can also significantly improve blood glucose, liver function indicators and hepatic steatosis in mice, the effect is also better than that of single-target drugs, which further illustrates that the dual-target fusion protein provided herein can exert the pharmacodynamic effects of two targets at the same time.

### Example 10 The efficacy of repeated administration of GLP-1-Fc-GDF15 fusion protein in diet-induced obesity (DIO) mouse model

This example tested the effect of repeated administration of GLP-1-Fc-GDF15 fusion protein on appetite suppression, weight reduction, and improvement of various metabolic indicators in DIO mice. After four months of high-fat diet induction, C57BL/6 mice were randomly divided into groups according to body weight, and then subcutaneously administered with vehicle (PBS, twice a week), GLP-1-Fc-GDF15 fusion protein D13 (3 nmol/kg, twice a week; 10 nmol/kg, once a week) or Semaglutide (10 nmol/kg, once a day) for four weeks. At the same time, a parallel control of normal mice was performed. During the test, changes of body weight and food intake were continuously monitored. At day 28 after the initial dose, autopsies were performed to detect various serum biochemical indicators and liver pathological indicators.

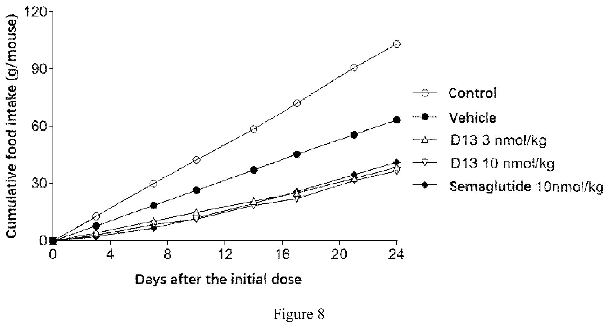

The results of body weight and food intake are shown in Figure 7, Figure 8 and Table 15; serum low-density lipoprotein cholesterol (LDL), ALT, AST and hepatic steatosis score parameters are shown in Table 16.

**Table 15 Effects of GLP-1-Fc-GDF15 fusion protein treatment on body weight and food intake of DIO mice**

| | Day 24 | |
|---|---|---|
| Group | Relative body weight change (%) | Cumulative food intake (g) |
| Vehicle | 3.77±0.83 | 63.5 |
| Semaglutide 10nmol/kg | -24.34±2.07*** | 41.2 |
| D13 3nmol/kg | -30.10±2.27*** | 38.6 |
| D13 10nmol/kg | -34.13±3.16***^{###} | 36.8 |

| | | |
|---|---|---|
| Note: Values represent the mean ± SEM of data from 7-8 animals in each group; ***-p<0.001, relative to vehicle; ^{###}-p<0.001, relative to Semaglutide; statistical analysis method: two-way ANOVA with Turkey's correction. | | |

**Table 16 GLP-1-Fc-GDF 15 fusion protein treatment improves blood biochemical indicators and hepatic steatosis in DIO mice**

| Group | LDL (mmol/L) | ALT (U/L) | AST (U/L) | Steatosis Score |
|---|---|---|---|---|
| Control | 0.265±0.027*** | 40.6±3.3*** | 118.5±9.9*** | 0.438±0.148*** |
| Vehicle | 1.280±0.057 | 261.4±41.0 | 279.6±32.2 | 2.857±0.143 |
| Semaglutide 10nmol/kg | 0.563±0.107*** | 41.8±7.3*** | 129.5±8.9*** | 1.125±0.206*** |
| D13 3nmol/kg | 0.573±0.076*** | 38.0±5.6*** | 114.9±12.0*** | 1.250±0.313*** |
| D13 10nmol/kg | 0.434±0.051*** | 29.7±2.5*** | 108.6±8.0*** | 0.750±0.189*** |

| | | | | |
|---|---|---|---|---|
| Note: Values represent the mean ± SEM of data from 7-8 animals in each group; ***-p<0.001, relative to vehicle; statistical analysis method: one-way ANOVA with Turkey's correction. | | | | |

The results show that the dual-target fusion protein D13 can significantly reduce the body weight and food intake in the DIO obese mouse model, and the effect is significantly better than Semaglutide at the same dose. At the same time, D13 can also significantly reduce low-density lipoprotein cholesterol, liver function indicators, and hepatic steatosis in mice, indicating that the dual-target molecule can be potentially used to treat obesity, non-alcoholic fatty liver disease, hyperlipidemia and other metabolic diseases.

The above represents just one preferred embodiment of the present invention, but the scope of protection of the present invention is not limited to this, and any person skilled in the art can easily think of changes or substitutions within the scope of the technology, shall be covered by the scope of protection of the present invention. Therefore, the scope of protection of the present invention shall be subject to the scope of protection of the claimed claims.

## Claims

1. A fusion protein, wherein the fusion protein comprises a first polypeptide fragment comprising a GLP-1 polypeptide; a second polypeptide fragment comprising an immunoglobulin Fc region; and a third polypeptide fragment comprising a GDF15 active domain; wherein the first polypeptide fragment, the second polypeptide fragment and the third polypeptide fragment are sequentially linked along the direction from amino terminus to carboxyl terminus through a linker peptide.

2. The fusion protein of claim 1, wherein the GLP-1 polypeptide comprises one of the following amino acid sequences: SEQ ID NO:1, SEQ ID NO:2, SEQ ID NO:3, or SEQ ID NO:4; or,
the GLP-1 polypeptide comprises an amino acid sequence having at least 85%, 90%, 95%, or 99% sequence identity to one of the following sequences: SEQ ID NO:1, SEQ ID NO:2, SEQ ID NO:3, or SEQ ID NO:4; or,
the GLP-1 polypeptide has no more than 6, 5, 4, 3, 2, 1 mutations relative to SEQ ID NO:1, SEQ ID NO:2, SEQ ID NO:3 or SEQ ID NO:4, while retaining the basic biological activity of the pre-mutation polypeptide.

3. The fusion protein of claim 2, wherein the GLP-1 polypeptide comprises one of the following mutations, or a combination of two or more mutations at different positions: A8G, G22E, I29V, A30E, A30Q, R36G;
preferably, the GLP-1 polypeptide comprises one of the following mutations: A8G, G22E, R36G; or A8G, G22E, A30E, R36G; or A8G, G22E, A30Q, R36G; or A8G, G22E, I29V, A30Q, R36G;
preferably, the GLP-1 polypeptide with mutations comprises one of the following amino acid sequences or an amino acid sequence having at least 85%, 90%, 95% or 99% sequence identity to one of the following sequences: SEQ ID NO: 5, SEQ ID NO: 6, SEQ ID NO: 7 or SEQ ID NO: 8.

4. The fusion protein of claim 1, wherein the immunoglobulin Fc region is selected from one of IgG1 Fc, IgG2, IgG3 Fc, and IgG4 Fc;
preferably, the immunoglobulin Fc region is selected from one of IgG1 Fc and IgG4 Fc;
preferably, the immunoglobulin Fc region is a human immunoglobulin Fc region.

5. The fusion protein of claim 4, wherein the immunoglobulin Fc region is a native immunoglobulin Fc region or an immunoglobulin Fc region variant.

6. The fusion protein of claim 5, wherein the immunoglobulin Fc region comprises an amino acid sequence of SEQ ID NO: 9 or SEQ ID NO: 10; or, the immunoglobulin Fc region comprises an amino acid sequence having at least 85%, 90%, 95% or 99% sequence identity to one of the following sequences: SEQ ID NO: 9, SEQ ID NO: 10; or, the immunoglobulin Fc region has no more than 6, 5, 4, 3, 2, 1 mutations relative to SEQ ID NO: 9, SEQ ID NO: 10, while retaining the basic biological activity of the pre-mutation polypeptide.

7. The fusion protein of claim 5, wherein the immunoglobulin Fc region variant comprises the following mutation: amino acid at position 356 of IgG Fc is substituted with an amino acid other than aspartic acid (D), glutamic acid (E) and cysteine (C) according to EU numbering;
preferably, the amino acid at position 356 of IgG Fc is substituted with one of the followings according to EU numbering: glycine (G), serine (S), alanine (A), threonine (T), valine (V), asparagine (N), leucine (L), isoleucine (I), glutamine (Q), tyrosine (Y), phenylalanine (F), histidine (H), proline (P), methionine (M), lysine (K) and arginine (R);
more preferably, the amino acid at position 356 of IgG Fc is substituted with one of the followings according to EU numbering: alanine (A), glutamine (Q), arginine (R), asparagine (N).

8. The fusion protein of claim 5, wherein the immunoglobulin Fc region variant comprises the following mutation: amino acid at position 439 of IgG Fc is substituted with an amino acid other than arginine (R), histidine (H), lysine (K), and cysteine (C) according to EU numbering;
preferably, the amino acid at position 439 of IgG Fc is substituted with one of the followings according to EU numbering: glycine (G), serine (S), alanine (A), threonine (T), valine (V), aspartic acid (D), asparagine (N), leucine (L), isoleucine (I), glutamic acid (E), glutamine (Q), tyrosine (Y), phenylalanine (F), proline (P), methionine (M);
more preferably, the amino acid at position 439 of IgG Fc is substituted with one of the followings according to EU numbering: alanine (A), aspartic acid (D), asparagine (N), glutamic acid (E), glutamine (Q).

9. The fusion protein of claim 5, wherein the immunoglobulin Fc region variant further comprises the following mutations: the amino acids at positions 234 and 235 of IgG Fc are substituted with alanine (A) respectively, and/or the amino acid at position 447 is deleted according to EU numbering.

10. The fusion protein of claim 5, wherein the immunoglobulin Fc region variant comprises one of the following amino acid sequences, or an amino acid sequence having at least 85%, 90%, 95% or 99% sequence identity to one of the following sequences: SEQ ID NO: 11-37.

11. The fusion protein of claim 1, wherein the GDF15 active domain comprises an amino acid sequence selected from one of the following groups:
SEQ ID NO: 38; or,
an amino acid sequence having at least 85%, 90%, 95% or 99% sequence identity to SEQ ID NO: 38, or,
1-14 amino acid truncations at the N-terminus of SEQ ID NO: 38, and/or SEQ ID NO: 38 having no more than 5, 4, 3, 2, 1 mutations while retaining the basic biological activity of the pre-mutation polypeptide.

12. The fusion protein of claim 11, wherein the position of amino acid mutation in SEQ ID NO: 38 is selected from one, two or three of the followings: position 5, position 6, position 21, position 26, position 30, position 47, position 54, position 55, position 57, position 67, position 69, position 81, position 94, position 107;
preferably, the amino acid mutation in SEQ ID NO: 38 is selected from one, any two or three of the different positions of the followings: D5E, H6D, H6E, R21Q, R21H, D26E, A30S, A47D, A54S, A55E, M57T, R67Q, K69R, A81S, T94E, K107Q.

13. The fusion protein of claim 11, wherein the GDF15 active domain comprises one of the following amino acid sequences, or an amino acid sequence having at least 85%, 90%, 95% or 99% sequence identity to one of the following sequences: SEQ ID NO: 38-58.

14. The fusion protein of claim 1, wherein the linker peptide comprises a first linker peptide and a second linker peptide;
preferably, each of the first linker peptide and the second linker peptide independently comprises amino acid sequences selected from the followings: SEQ ID NO: 78-81;
more preferably, the N-terminus of the first linker peptide is connected with the C-terminus of the GLP-1 polypeptide, the C-terminus of the first linker peptide is connected with the N-terminus of the immunoglobulin Fc region, the N-terminus of the second linker peptide is connected with the C-terminus of the immunoglobulin Fc region, and the C-terminus of the second linker peptide is connected with the N-terminus of the GDF15 active domain.

15. The fusion protein of claim 1, wherein the fusion protein comprises one of the following amino acid sequences, or an amino acid sequence having at least 85%, 90%, 95% or 99% sequence identity to one of the following sequences: SEQ ID NO: 59-77.

16. A homodimeric fusion protein comprising the fusion protein of any one of claims 1-15.

17. A biological material, wherein the biological material is any one of (i), (ii), and (iii):
(i) a nucleic acid comprising a nucleotide sequence encoding the fusion protein of any one of claims 1-15;
(ii) a vector comprising the nucleic acid of (i);
(iii) a host cell comprising the nucleic acid of (i) and/or the vector of (ii).

18. A method of preparing the fusion protein of any one of claims 1-15 comprising culturing the host cell of claim 17 under conditions suitable for expressing the fusion protein, and purifying the fusion protein expressed by the host cell.

19. A pharmaceutical composition comprising the fusion protein of any one of claims 1-15 and/or the homodimeric fusion protein of claim 16 as an active ingredient, and the fusion protein and/or the homodimeric fusion protein are present in the pharmaceutical composition in a therapeutically effective amount.
preferably, the pharmaceutical composition also comprises a pharmaceutically acceptable carrier.

20. Use of the fusion protein of any one of claims 1-15, the homodimeric fusion protein of claim 16, or the pharmaceutically composition of claim 19 in any of the following (iv) and (v):
(iv) in the manufacture of a medicament for treating metabolic diseases, preferably, the metabolic diseases comprise type II diabetes, obesity, dyslipidemia, diabetic nephropathy, nonalcoholic steatohepatitis, nonalcoholic fatty liver disease;
(v) in the manufacture of a medicament for reducing food intake, body weight, insulin level, triglyceride level, cholesterol level or glucose level in a subject.

21. The fusion protein of any one of claims 1-15, the homodimeric fusion protein of claim 16, the pharmaceutical composition of claim 19 for use in treating metabolic diseases or reducing food intake, body weight, insulin level, triglyceride level, cholesterol level or glucose level in a subject;
preferably, the metabolic diseases comprise type II diabetes, obesity, dyslipidemia, diabetic nephropathy, nonalcoholic steatohepatitis, nonalcoholic fatty liver disease.

22. A method of treating metabolic diseases or reducing food intake, body weight, insulin level, triglyceride level, cholesterol level or glucose level in a subject comprising administering to the subject the fusion protein of any one of claims 1-15, the homodimeric fusion protein of claim 16, or the pharmaceutical composition of claim 19;
preferably, the metabolic diseases comprise type II diabetes, obesity, dyslipidemia, diabetic nephropathy, nonalcoholic steatohepatitis, nonalcoholic fatty liver disease.
